# EUROPEAN PATENT APPLICATION

(11) **EP 1 493 446 A1**
(43) Date of publication of application: **05.01.2005**
(21) Application number: 04005142.7
(22) Date of filing: 23.09.1994
(51) Int. Cl.: A61K 38/17, C07K 14/435, C07K 14/705, C12N 15/12, C12P 21/00, C07K 14/47

(54) **Modified truncated complement system regulators**

(30) Priority: 24.09.1993 US 126505
(62) Divisional of application: 94929330.2
(71) Applicant: WASHINGTON UNIVERSITY, St. Louis, MO 63130 (US)
(72) Inventor: Atkinson, John P., St. Louis Missouri 63122 (US); Hourcade, Dennis, Creve Coeur Missouri 63146 (US); Krych, Malgorzata, St. Louis Missouri 63108 (US)
(74) Representative: HOFFMANN - EITLE

(57) **Abstract**

Analogs of regulators of complement activation (RCA) proteins which have altered specifities and affinities for the targets C3b and/or C4b are described. These analogs are obtained by substituting amino acids which affect the complement inhibitory activities of these proteins, substituting, rearranging or adding SCRs or SCR regions to the proteins, deleting amino acid or adding SCRs or SCR regions to the proteins, deleting amino acid sequences, and combinations thereof.

## Description

### Background of the Invention

The invention relates to modified and/or shortened forms of complement regulators derived from regulatory proteins of complement activation (RCA), especially CR1.

The complement system serves to aid in the removal of foreign substances and of immune complexes from animal hosts. This system and its regulation is reviewed by Hourcade, D., et al., Advances in Immunol. (1989) 45:381-416. Briefly, the complement system generates, either by a "classical pathway" or an "alternative pathway," C3b which binds to target immune complexes or foreign substances and marks them for destruction or clearance. C3b is generated from its precursor C3 by the proteolytic enzymes collectively designated "C3 convertase." One form of C3 convertase is generated in the classical pathway by the association of the proteins C4b and C2a. The other form is generated in the alternative pathway by association of C3b and Bb. Both C3 convertases can associate with an additional C3b subunit to form the C5 convertases, C3bBbC3b and C4bC2aC3b, both of which are active in the production of the C5-C9 membrane attack complex which can cause cell lysis, and the production of C5a, a major pro-inflammatory agent.

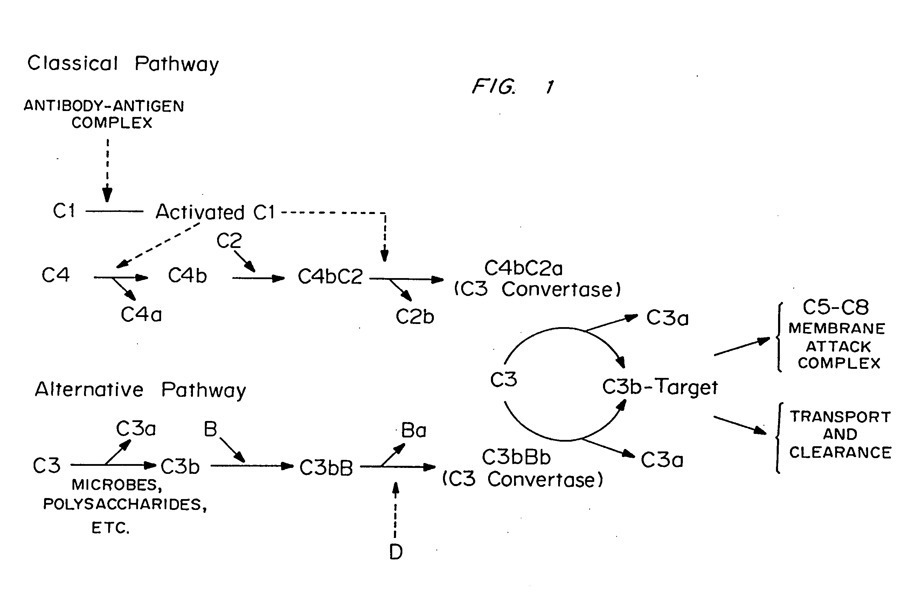

Both C3b, and less directly, C4b, are agonists in the complement system. This is shown in the diagram in Figure 1.

The complement system is regulated via a number of interrelated mechanisms. There are two general mechanisms for inhibition of the destructive components of the complement system. The first mechanism is generally reversible, facilitating the dissociation of the C3 convertases--i.e., C3b from Bb and C4b from C2a. Facilitation of dissociation is sometimes known as decay acceleration. The dissociation may also involve reversible binding of the antagonist proteins to C3b or C4b components, thus preventing their reassociation. The other mechanism, which is an irreversible inactivation process, results from proteolytic cleavage of the C3 convertase components C3b or C4b by the serine protease factor I. This proteolytic cleavage occurs only in the presence of a cofactor. Both general regulatory mechanisms, the facilitation of dissociation of C3b and C4b and the inactivation of C3b and C4b through cleavage by factor I, also apply to the inhibition of the alternative pathway C5 convertase (C3bBbC3b) and the classical pathway C5 convertase (C4bC2aC3b).

The proteins encoded by a region of the genome which is designated the "regulators of complement activation" (RCA) gene cluster are involved in both of the foregoing mechanisms. Currently, it is known that at least six complement proteins are encoded by this region. These are summarized in Table 1.

**Table 1**

| **RCA Proteins: Functional Profile** | | | |
|---|---|---|---|
| **Name** | **Primary Ligand(s)** | **Decay Acceleration (Dissociation)** | **Cofactor Activity** |
| | | | |
| CR1 | C3b/C4b | + | + |
| MCP | C3b/C4b | - | + |
| DAF | C3b/C4b C3 Convertases | + | - |
| C4bp | C4b | + | + |
| Factor H | C3b | + | + |
| CR2 | C3dg | - | ND |

These proteins share certain structural similarities which are further described below.

The reversible binding to C4b or C3b to dissociate the C3 convertases is effected by two plasma proteins designated C4 binding protein (C4bp) and factor H, and by two membrane proteins designated decay acceleration factor (DAF) and complement receptor 1 (CR1). Reversible binding to C4b is effected by C4bp, DAF and CR1 while reversible binding to C3b is effected by factor H, DAF and CR1.

The irreversible inactivation of the C3 convertases resulting from proteolytic cleavage of convertase components C3b or C4b by the enzyme factor I can occur by virtue of cofactor activity effected by the above-mentioned factor H and C4bp in the plasma and by CR1 and membrane cofactor protein (MCP) at the cell surface. Cofactor activity for cleavage of C3b is effected by factor H, CR1 and MCP while cofactor activity for cleavage of C4b is effected by C4bp, CR1 and MCP. It is also possible that the sixth protein, complement receptor 2 (CR2), has this cofactor activity at the cell surface.

In summary, of the six proteins encoded by the RCA gene cluster, factor H, C4bp, and CR1 have both reversible dissociation activity and irreversible cofactor activity; DAF has only reversible dissociation activity, and MCP and possibly CR2 have only irreversible cofactor activities. CR1, DAF and MCP interact with both C3b and C4b; C4bp interacts primarily with C4b, and factor H interacts primarily with C3b.

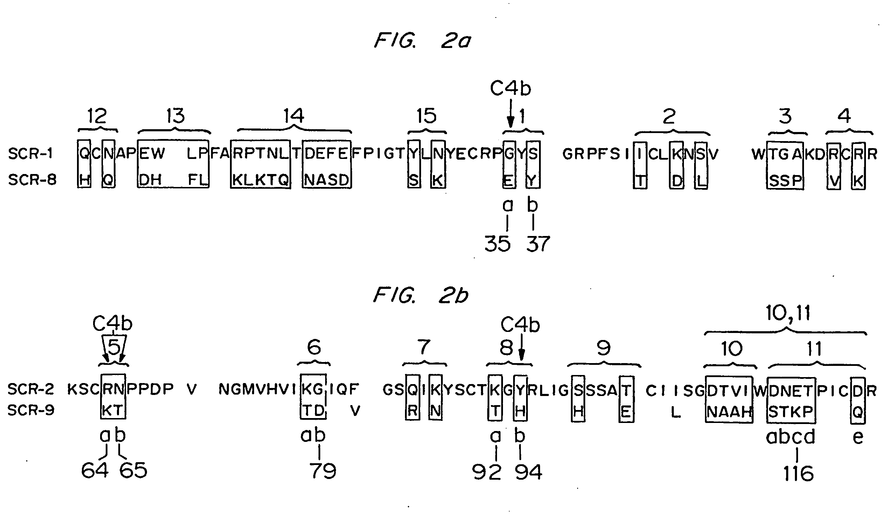

The cDNAs corresponding to CR1, CR2, DAF, MCP, C4bp, and factor H have all been obtained and sequenced. Evaluation of these comparative sequences has lead to the alignment set forth in Figure 2A which shows the organization of the RCA proteins into short consensus repeat ("SCR") - containing and non-SCR-containing regions with the N-terminal ends at the left. In this figure, TM refers to transmembrane domain, C to cytoplasmic domain, 0 to 0-linked glycosylation domain, G to glycolipid anchor, U to domain with unknown significance and D to a disulfide bridge-containing domain.

There is considerable uniformity among the RCA family of proteins. All of them are composed of 60-70 amino acid repeating units commonly designated "short consensus repeats" (SCRs). Each SCR shares a number of invariant or highly conserved amino acid residues with other SCRs in the same protein or SCRs in other family members. Those members of the family which are membrane bound also have at their C termini either transmembrane regions and intracellular regions or a glycolipid anchor.

The SCRs form the extracellular portions of those members of the family which are membrane-bound and almost all of the protein structure in the secreted members. Two covalently-crosslinked cysteine pairs establish two loops within each SCR. The smallest family members are DAF and MCP; each contains four SCRs followed by an 0-linked glycosylation region. DAF is terminated with a glycolipid anchor while MCP ends with an extracytoplasmic segment of unknown significance, a transmembrane region and an intracellular domain. Of the secreted members of the family, factor H contains twenty SCRS, while the native form of C4bp is an association of seven subunits of eight SCRs (the C4bp alpha chains) and one subunit of three SCRs (the C4bp beta chain). Both C4bp chains conclude with non-SCR domains that interconnect the chains through disulfide linkages. CR2 contains sixteen SCRs, a transmembrane region and an intracellular domain. The most common polymorphic form of CR1 contains four repeating units of seven similar SCRs (long homologous repeats or LHRS) numbered 1-28, followed by an additional two SCRs designated 29 and 30, a transmembrane region and an intracellular region.

Klickstein, L.B., et al., J. Exp. Med. (1988) 168:1699-1717, described the identification of distinct C3b and C4b recognition sites in CR1 using deletion mutagenesis. They concluded that a single primary C4b binding site is located in SCR 1-2 (Sequence ID Nos. 1 and 3), while two major C3b binding sites are located in SCRs 8-9 (Sequence ID Nos. 2 and 4) and SCR 15-16. C3b cofactor activity was localized to SCR 8-9 and SCR 15-16. More recently it has been shown the CR1 active site containing SCR 8-9 extends to SCR 10, and by analogy, the active site that contains SCR 15-16 (which is only one amino acid different than SCR 8-9) must extend to SCR 17. (Kalli, et al., J. Exp. Med. 174, 1451-1460 (1991); Makrides, et al., J. Biol. Chem. 267, 24754-24761 (1992)). The CR1 active site containing SCR 1-2 extends to SCR 3 and/or 4, as reported by Makrides, et al., (1992).

The murine C4bp binding site, and presumably the C4b cofactor and C4bC2a decay acceleration active sites, was reported to extend from SCRs 1-3 in the alpha chain by Ogata, et al., J. Immunology 150, 2273-2280 (1993).

The factor H binding site, and probably the C3b cofactor and C3bBb decay acceleration active sites, lies within the first five SCRs. The CR2 binding site for C3b proteolytic products extends through the first two SCRs, Kalli, et al., J. Immunology 147: 509-594 (1991); Carel, et al., J. Biol. Chem. 265: 12293-12299 (1990).

The MCP active sites extend through all four SCRs: SCRs 2-4 are required for C3b and C4b cofactor activity. SCR 1 appears unnecessary for C3b cofactor activity and binding but appears necessary for efficient C4b cofactor activity and binding, as reported by Adams, et al., J. Immunology 147:3005-3011 (1991). The DAF active sites extend through SCRs 2-4, as reported by Coyne, et al., J. Immunology 149: 2906-2913 (1992).

Hourcade, D., et al., J. Exp. Med. 168:1255-1270 (1988), described a cDNA clone designated CR1-4 that encodes the first eight and one-half amino terminal SCRs of CR1. This cDNA was transfected into COS cells which resulted in the synthesis of a secreted truncated form of CR1 with a molecular weight of 78 kd (Krych, M. et al., Proc. Natl. Acad. Sci. USA 88:4353-4357 (1991). This shortened form of the protein, as shown herein below, binds mainly C4b. This shortened form has now been determined to have C4b cofactor activity, as described herein.

The multiple binding sites of CR1 can cooperate in their interactions with C3b-containing targets. *In vitro*, CR1 binds C3b-C3b dimers much more tightly than C3b monomers because binding to dimers can occur simultaneously at two sites in the same CR1 molecule, as reported by Wong and Farrell, J. Immunol. (1991) 146:656; Ross and Medof Adv. Immunol. (1985) 37:217). Deletion of one of the two primary C3b binding sites can reduce the binding of CR1 to C3b-C3b by a factor of ten, as reported by Wong and Farrell, J. Immunol. (1991) 146:656. It is likely that the primary C4b binding site also cooperates with the primary C3b binding sites in interactions with targets that contain both C3b and C4b. These effects have an important consequence *in vivo*: CR1 has a higher affinity for targets densely coated with C3b and with targets densely coated with C3b plus C4b.

The C5 convertases, which are important in the stimulation of inflammation and in lysis of some target cells, are composed of multiple CR1 ligands: The classical C5 convertase contains C3b and C4b (C4bC3bC2a) while the alternative pathway C5 convertase contains two C3b proteins (C3bC3bBb). Inactivation of the C5 convertases by CR1 can also involve cooperation between more than one CR1 binding site. Wong and Farrell. J. Immunol. (1991) 146:656 showed that more than one CR1 C3b binding site may be essential for effective inhibition of alternative pathway C3 and C5 convertases.

The proteins encoded by the RCA gene cluster can be prepared recombinantly and used in diagnosis and therapy for the regulation of the complement system. The problems of transplantation of xenografts are reviewed by Platt, J.L., et al., in Immunology Today (1990) 11:450-457. Evidence has accumulated that the immediate hyperacute rejection of discordant xenografts is caused by recipient complement activity. Transgenic animals expressing human complement regulators (such as DAF or MCP) on cell surfaces could be an abundant source of organs that would be protected from hyperacute rejection in human recipients. A soluble complement inhibitor could also play a role in protecting xenografts from complement-mediated rejection.

The ability of a recombinant soluble form of CR1 to inhibit inflammation in the reversed passive Arthus reaction in rats was described by Yeh, C.G., et al., J. Immunol (1991) 146:250-256. This soluble CR1 was obtained from Chinese hamster ovary (CHO) cells expressing a CR1 genetic construct which had been mutated to remove the transmembrane and cytoplasmic domains. The ability of a similar soluble CR1, produced recombinantly in CHO cells, to inhibit post-ischemic myocardial inflammation and necrosis in rats was reported by Weissman, H.F., et al., Science (1990) 249:146-151.

Proteins related to the RCA proteins have also been shown to be produced by viruses, presumably as a mechanism whereby infection by the virus can be facilitated, as reported by Kotwaal, J., et al., Nature (1988) 335:176-178; McNearney, T.A., J Exp Med (1987) 166:1525-1535.

Complete inhibition of the complement system on a long-term basis is not likely to be desirable in most individuals. In some cases of autoimmune disease, inhibition of the classical pathway alone may be sufficient. In the case of the xenograft transplants, however, stringent inhibition of both pathways may be important. Similar stringency may be required for other applications. Accordingly, alternative modulators of the complement system with regulatable binding activities would be desirable.

It is therefore an object of the present invention to provide modified complement regulators which can be administered in soluble form for treatment of inflammatory disorders or to reduce an individual's ability to reject foreign materials.

It is a further object of the present invention to provide modified complement regulators which are shorter and more easily and economically produced than the more complex naturally occurring proteins.

It is another object of the present invention to provide complement regulators which combine the activities of different complement regulators to provide enhanced capability of inhibiting complement proteins specifically and systemically, both in the classical and alternative pathways.

### Summary of the Invention

Analogs of RCA-encoded proteins which are modified full-length or truncated forms of these regulatory proteins are described and demonstrated to inhibit complement. These analogs include RCA proteins having site-specific mutations, RCA protein hybrids which contain one or more SCRs from more than one RCA protein, modifications of RCA recombinants in which SCRs are arranged in different orders, and truncated versions of RCA proteins containing as few three SCRs. The modified proteins retain complement inhibitory activity, which may be altered in specificity, affinity, or mechanism.

The modified proteins are demonstrated by the ΔSCR11, subst1 mutation of CR1-4(8,9) which carries only three complete SCRs (amino acids 1-194, SCR 1-3) and has C3b activity; the 1ar,5r,8br mutation of CR1-4(8,9) which has accentuated C3b and C4b activities; a three SCR form composed of the first 194 amino acids of 1ar,5r,8br; mutants 10, 10, 10,11, 11c, 8a, 6b, 14, and 15, which are mutations of CR1-4 with enhanced activities; and CR1-4 stop7, CR1-4(8,9) stop7, and 1ar,5r,8br stop 7, which are all shorter (7 SCR) forms of their respective parent proteins (CR1-4, CR1-4(8,9) and 1ar,5r,8br, respectively).

These analogs are useful in controlling the complement system, and thus may be useful in the treatment of autoimmune diseases, the suppression of rejection of transplants, in diagnosis and the reduction in tissue damage associated with myocardial infarctions and cerebral vascular accidents. They may also play a role in the diagnosis of conditions associated with complement activation and immune complex formation.

### Brief Description of the Drawings

Figure 1 is a schematic of the classical and alternate complement pathways.
Figures 2A and 2B show schematically the structures and relationships of RCA-encoded proteins. Figure 2A is the short consensus repeat shown with highly conserved residues.
Figure 2B is a schematic of the SCR organization of the RCA proteins: CR1, CR2, DAF, MCP, C4 binding protein, and factor H.
Figures 3A and 3B compare the amino acid sequences of SCR-1 and SCR-8 (Sequence ID Nos. 1 and 2, respectively) (Figure 3A) and SCR-2 and SCR-9 (Sequence ID Nos. 3 and 4, respectively) (Figure 3B) of full-length CR1. The amino acid sequences of SCR 15-16 are identical to those of SCR 8-9, with the exception of a T at position 110. The enumeration is consistent with that of Hourcade et al., J. Exp. Med. (1988) 168:1255-1270, with the first amino acid (Q) of the mature receptor designated as amino acid 1. The corresponding position in CR1 SCR-8 is similarly designated.

### Detailed Description of the Invention

Described herein is a family of RCA protein analogs which can be used to modulate the complement system. The analogues can be used to alter the binding specificity of the members of this protein family in both membrane-bound and soluble forms.

As used herein, "RCA proteins" refers to proteins encoded by the gene cluster at the genetic region 1q32, which encodes six known proteins effective in complement regulation: factor H, C4bp, CR1, CR2, DAF and MCP. In addition, apparent coding regions similar to the amino terminal coding region of the CR1 gene and the MCP gene have been located in this cluster, although it is unclear whether these sequences are expressed (Hourcade, D., et al., J. Biol. Chem. 265:974-980 (1990); Hourcade, D., et al., Genomics 12:289-300 (1992)). The term "RCA proteins" also refers to a group of proteins which include short consensus repeats (SCR) which are homologous to SCR in CR1, CR2, MCP. DAF, factor H, and C4bp.

The modified proteins described herein are collectively referred to as "modified RCA proteins". These include truncated, hybrid and recombined forms of the RCA proteins. "Truncated" proteins are shorter versions of the RCA proteins, typically modified so as to remove the C-terminal regions which effect membrane binding or secretion and sometimes modified further by deletion of one or more SCRS. "Hybrid" proteins are RCA proteins that are composed of portions, i.e., the SCRS, of one RCA protein combined with SCRs of one or more other RCA proteins. "Recombined" forms are those wherein the SCRs of an RCA protein are rearranged in a new order. Also included are proteins having site specific substitutions and deletions of one or more amino acids. "Modified RCA proteins" include proteins which result from combinations of these changes.

The short consensus repeat sequence is shown in Figure 2A. As shown schematically in Figure 2B, CR1 contains 30 SCR, followed by transmembrane and cytoplasmic regions. CR2 contains 16 SCR, followed by transmembrane and cytoplasmic regions. DAF and MCP each contain four SCR, followed by glycolipid anchor regions. The C4 binding protein is a more complex protein, having seven alpha subunits and one beta subunit. The alpha subunit consists of eight SCR and the beta subunit consists of three SCR. Factor H consists of twenty SCR.

In some embodiments, modifications are made using corresponding SCRs of the protein as sites for alteration. By "corresponding SCR" is meant the most highly homologous SCR as judged by the amino acid sequences of the protein. Exon structure can in some cases facilitate this assignment. As noted above, SCRs 1-3 of CR1 correspond to SCRs 2-4 of DAF. SCRs 1-3 of factor H, CR1, C4bp and MCP are corresponding sequences among these proteins. CR1 is organized into a series of long homologous repeats (LHRS) containing 7 SCRs so that CR1 SCRs 1-7 correspond to CR1 SCRs 8-14; 15-21; and 22-28. CR2 is organized into a series of long homologous repeats of 4 SCRs in length. SCRs 1-2 of CR1 correspond to SCRs 3-4, SCRs 7-8, SCRs 11-12 and SCRs 15-16 of CR2.

These proteins are characterized by C4b- binding activity, C3b-binding activity, C4b cofactor activity, and C3b cofactor activity. In general, it takes two to three SCRs for each activity. Activities which are biologically important include decay acceleration or dissociation, C3b cofactor activity and C4b cofactor activity. Cofactor activity requires binding but binding alone may not be sufficient for cofactor activity.

It is generally accepted that CR1 C4b binding and cofactor activity requires SCRs 1, 2 and 3, 8, 9, and 10, or 15, 16, and 17, which are corresponding regions of the protein. C3b binding and cofactor activity requires SCRs 8, 9, and 10, or 15, 16, and 17, which are corresponding regions of the protein. MCP C4b binding and cofactor activity requires SCRs 1, 2, 3, and 4. MCP C3b binding requires SCRs 3 and 4; cofactor activity requires SCRs 2, 3, and 4. C4bp C4b binding and cofactor activity requires SCRs 1, 2, and 3. DAF decay accelerating activity requires SCRs 2, 3, and 4. Factor H C3b binding activity is mediated by the first five SCRs.

Based on these discoveries, it is possible to design a more potent soluble complement inhibitor by modifying corresponding regions to increase affinity for C4b and C3b or to design soluble complement inhibitors that specifically inhibit one part of the complement system. These modifications can be in the form of specific substitutions of amino acids that alter C3b or C4b binding within corresponding SCRs of CR1 or other RCA proteins, or substitution of SCRs from one protein into another.

### Substitution of SCR regions from one recrulatory protein into a second regulatory protein.

The identification of the amino acid sequences essential (or refractory) to binding to C4b and C3b and C4b and C3b cofactor activity permits transposition of similar sequences into corresponding regions of the same protein or corresponding regions of other family members or alteration of sequences which bind C3b and C4b so as to alter their affinities. Corresponding regions have been identified by degree of amino acid sequence homology.

In the case of CR1, four corresponding regions of interest are SCRs 1-3, SCRs 8-10, SCRs 15-17 and SCRs 22-24. The SCR portions labeled 2-4 in Figure 2B for DAF correspond to those labeled 1-3, 8-10, 15-17 and 22-24 for CR1 in Figure 2B. Substitution of portions of DAF with homologous CR1 sequences provides forms of DAF with cofactor activity and/or binding activity, such as is exhibited by CR1. Similarly, substitutions of portions of MCP with homologous sequences provides forms of MCP with increased binding affinity and cofactor activity and/or increased dissociation activity.

### Specific Amino Acid Substitutions

### Addition of Binding Sites

Specific amino acids are selected for substitution based on studies that elucidate their roles in complement regulation in specific active sites. Substitution can be employed in order to alter the activity of additional RCA active sites in the same or other proteins. In this manner, binding and cofactor sites can be added to SCRs not normally contributing directly to binding capacity.

The standard one letter abbreviations for amino acids are used herein.

For example, the C3b and C4b binding and cofactor sequence in CR1, N-A-A-H-W-S-T-K-P-P-I-C-Q (amino acids 49-61 of Sequence ID No. 4), can be transferred to corresponding locations or to locations referenced to conserved amino acids in alternative SCRs to confer C3b binding. Conversely, the homologous sequence in SCR-2 of CR1, i.e., D-T-V-I-W-D-N-E-T-P-I-C-D (amino acids 49-61 of Sequence ID No. 3), can be transferred by substitution to other locations in C3b-binding SCRs in order to decrease C3b and C4b binding and cofactor activity. The C4b binding regions are shown to be associated with three separate critical locations in SCR-1 and SCR-2 of CR1 in the proximity of amino acids 35, 64-65, and 92-94. Alterations in amino acid sequences of the corresponding SCRs in CR1 or in additional RCA family members or their truncated, hybrid, or recombined forms in these positions alter C4b binding and cofactor activities and in at least one case alter C3b binding and cofactor activities.

### Substitution of Similar Amino Acids

Structurally similar amino acids can be substituted in such transfers for some of the specified amino acids. Structurally similar amino acids include: (I,L,V); (F,Y); (K,R); (Q,N); (D,E); and (G,A).

### Deletion of Amino Acids

It also may be advantageous to delete amino acids from specific active sites in order to alter or enhance complement regulatory activity.

### Construction of Truncated Forms

In some embodiments, it will be advantageous to delete a specific activity by deletion of a region known to have a particular activity. It may also be desirable to delete the region of the protein which anchors the naturally occurring protein to the cell surface, for example, the transmembrane and cytoplasmic regions or the glycolipid anchor region.

### Modifications which enhance Cofactor Activity

In general, either C3b or C4b cofactor activity can be enhanced by substitutions which increase the binding activity of the other factor, i.e., to increase C4b cofactor activity, amino acids are substituted into the modified protein which increase C3b binding and *vice versa.* This is demonstrated in the examples, specifically by the mutants shown in Table 2. An example of a single amino acid substitution that enhances both C4b cofactor activity and C3b cofactor activity is CR1 mutant 6b: changing the G at 79 to D increases C4b cofactor, as well as C3b binding and cofactor activity.

### Preparation of the Analogs

The modified proteins described herein are most conveniently prepared using recombinant techniques, although in some cases they can be prepared by enzymatic cleavage, for example, to yield truncated or soluble forms of the naturally occurring proteins. The genes encoding the various members of the RCA protein family are of known sequence and are published.

cDNA encoding CR1 has been described by Klickstein, L.B., et al., J. Exp. Med. (1987) 165:1095, Klickstein, L.B., et al., J. Exp. Med. (1988) 168:1699; Hourcade, D., et al., J. Exp. Med. (1988) 168:1255, the teachings of which are incorporated by reference.

The cDNA encoding CR2 has been described by Moore, M.D., et al., Proc. Natl. Acad. Sci. USA (1987) 84:9194, and by Weiss, J.J., et al., J. Exp. Med. (1988) 167:1047, the teachings of which are incorporated by reference.

The cDNA encoding DAF has been described by Caras, I.W., et al., Nature (1987) 325:545, and by Medof, M.E., et al., Proc. Natl. Acad. Sci. USA (1987) 84:2007, the teachings of which are incorporated by reference.

The cDNA encoding MCP has been described by Lublin, D.M., et al., J. Exp. Med. (1988) 168:181, the teachings of which are incorporated by reference.

The cDNa encoding the C4bp alpha chain has been described by Chung, L.P, et al., Biochem. J. (1985) 230:133, and the cDNA encoding the C4bp beta chain has been described by Hillarp, A., and Dahlback, B., Proc. Natl. Acad. Sci. USA (1990) 87:1183, the teachings of which are incorporated by reference.

The cDNA encoding factor H has been described by Ripoche, J., et al., Biochem. J. (1988) 249:593, the teachings of which are incorporated by reference.

Since the cDNAs encoding these proteins are known and the amino acid sequences have been deduced, comparison of corresponding regions of the various proteins of the member families has been possible. In addition, the availability of the cDNA sequence makes possible the preparation of genetic constructs encoding truncated forms and other modified forms of the proteins using standard site-directed mutagenesis techniques, such as those described by Kunkel, T.A., et al., Methods Enzymol. (1987) 154:367-382.

Accordingly, the first step in the preparation of the analogs requires identification of the corresponding region of the target protein through sequence homology and site-directed mutagenesis in this region of the gene to alter C4b or C3b binding properties.

After the gene encoding the analog is prepared, the modified gene is expressed using standard recombinant techniques. The gene sequence is ligated into a suitable expression vector under the control of sequences known to be appropriate to the desired host. Production of recombinant proteins in microbial systems such as *E. coli, B. subtilis,* various strains of yeasts, and other fungi, such as *Aspergillus,* is well known. It may be advantageous to produce the desired analogs in cells of higher organisms as well, such as the standard BPV/C127 system, the Baculovirus/insect cell system, CHO cells, COS cells, and other mammalian cells, or in transgenic animals. Standard expression systems in various cell lines are well known and standard in the art.

Transgenic animals can be constructed for several species. The gene is placed under the control of a suitable promoter, for example, the metallothionine promoter or a tissue specific promoter, and the gene microinjected into an embryo, which is then implanted into a surrogate mother. Production in transgenic animals is important in the context of preparing transplants for use in other species.

### Construction of Transgenic Animals.

### Animal Sources

Animals suitable for transgenic experiments can be obtained from standard commercial sources. These include animals such as mice and rats for testing of genetic manipulation procedures, as well as larger animals such as pigs, cows, sheep, goats, and other animals that have been genetically engineered using techniques known to those skilled in the art. These techniques are briefly summarized below based principally on manipulation of mice and rats.

### Microinjection Procedures

The procedures for manipulation of the embryo and for microinjection of DNA are described in detail in Hogan et al. Manipulating the mouse embryo, Cold Spring Harbor Laboratory, Cold Spring Harbor, NY (1986), the teachings of which are incorporated herein. These techniques are readily applicable to embryos of other animal species, and, although the success rate is lower, it is considered to be a routine practice to those skilled in this art.

### Transgenic Animals

Female animals are induced to superovulate using methodology adapted from the standard techniques used with mice, that is, with an injection of pregnant mare serum gonadotrophin (PMSG; Sigma) followed 48 hours later by an injection of human chorionic gonadotrophin (hCG; Sigma). Females are placed with males immediately after hCG injection. Approximately one day after hCG, the mated females are sacrificed and embryos are recovered from excised oviducts and placed in Dulbecco's phosphate buffered saline with 0.5% bovine serum albumin (BSA; Sigma). Surrounding cumulus cells are removed with hyaluronidase (1 mg/ml). Pronuclear embryos are then washed and placed in Earle's balanced salt solution containing 0.5% BSA (EBSS) in a 37.5°C incubator with a humidified atmosphere at 5% CO₂, 95% air until the time of injection.

Randomly cycling adult females are mated with vasectomized males to induce a false pregnancy, at the same time as donor females. At the time of embryo transfer, the recipient females are anesthetized and the oviducts are exposed by an incision through the body wall directly over the oviduct. The ovarian bursa is opened and the embryos to be transferred are inserted into the infundibulum. After the transfer, the incision is closed by suturing.

### Embryonic Stem (ES) Cell Methods

### Introduction of cDNA into ES cells:

Methods for the culturing of ES cells and the subsequent production of transgenic animals, the introduction of DNA into ES cells by a variety of methods such as electroporation, calcium phosphate/DNA precipitation, and direct injection are described in detail in Teratocarcinomas and embryonic stem cells, a practical approach, ed. E.J. Robertson, (IRL Press 1987), the teachings of which are incorporated herein. Selection of the desired clone of transgene-containing ES cells is accomplished through one of several means. In cases involving sequence specific gene integration, a nucleic acid sequence for recombination with the gene of interest or sequences for controlling expression thereof is co-precipitated with a gene encoding a marker such as neomycin resistance. Transfection is carried out by one of several methods described in detail in Lovell-Badge, in Teratocarcinomas and embryonic stem cells, a practical approach, ed. E.J. Robertson, (IRL Press 1987) or in Potter et al Proc. Natl. Acad. Sci. USA 81, 7161 (1984). Calcium phosphate/DNA precipitation, direct injection, and electroporation are the preferred methods. In these procedures, a number of ES cells, for example, 0.5 X 10⁶, are plated into tissue culture dishes and transfected with a mixture of the linearized nucleic acid sequence and 1 mg of pSV2neo DNA (Southern and Berg, J. Mol. Appl. Gen. 1:327-341 (1982)) precipitated in the presence of 50 mg lipofectin in a final volume of 100 µl. The cells are fed with selection medium containing 10% fetal bovine serum in DMEM supplemented with an antibiotic such as G418 (between 200 and 500 µg/ml). Colonies of cells resistant to G418 are isolated using cloning rings and expanded. DNA is extracted from drug resistant clones and Southern blotting experiments using the nucleic acid sequence as a probe are used to identify those clones carrying the desired nucleic acid sequences. In some experiments, PCR methods are used to identify the clones of interest.

DNA molecules introduced into ES cells can also be integrated into the chromosome through the process of homologous recombination, described by Capecchi, (1989). Direct injection results in a high efficiency of integration. Desired clones are identified through PCR of DNA prepared from pools of injected ES cells. Positive cells within the pools are identified by PCR subsequent to cell cloning (Zimmer and Gruss, Nature 338, 150-153 (1989)). DNA introduction by electroporation is less efficient and requires a selection step. Methods for positive selection of the recombination event (i.e., neo resistance) and dual positive-negative selection (i.e., neo resistance and ganciclovir resistance) and the subsequent identification of the desired clones by PCR have been described by Joyner et al., Nature 338, 153-156 (1989) and Capecchi, (1989), the teachings of which are incorporated herein.

### Embryo Recovery and ES cell Injection

Naturally cycling or superovulated females mated with males are used to harvest embryos for the injection of ES cells. Embryos of the appropriate age are recovered after successful mating. Embryos are flushed from the uterine horns of mated females and placed in Dulbecco's modified essential medium plus 10% calf serum for injection with ES cells. Approximately 10-20 ES cells are injected into blastocysts using a glass microneedle with an internal diameter of approximately 20 µm.

### Transfer of Embryos to Pseudopregnant Females

Randomly cycling adult females are paired with vasectomized males. Recipient females are mated such that they will be at 2.5 to 3.5 days post-mating (for mice, or later for larger animals) when required for implantation with blastocysts containing ES cells. At the time of embryo transfer, the recipient females are anesthetized. The ovaries are exposed by making an incision in the body wall directly over the oviduct and the ovary and uterus are externalized. A hole is made in the uterine horn with a needle through which the blastocysts are transferred. After the transfer, the ovary and uterus are pushed back into the body and the incision is closed by suturing. This procedure is repeated on the opposite side if additional transfers are to be made.

### Identification of Transgenic Animals.

Samples (1-2 cm of mouse tails) are removed from young animals. For larger animals, blood or other tissue can be used. To test for chimeras in the homologous recombination experiments, i.e., to look for contribution of the targeted ES cells to the animals, coat color has been used in mice, although blood could be examined in larger animals. DNA is prepared and analyzed by both Southern blot and PCR to detect transgenic founder (F₀) animals and their progeny (F₁ and F₂).

Once the transgenic animals are identified, lines are established by conventional breeding and used as the donors for tissue removal and implantation using standard techniques for implantation into humans.

### Purification of Analogs

The analogs recombinantly produced in culture or animals can be purified from the cell culture using standard purification techniques such as chromatography, for example, immunoaffinity or ion-exchange chromatography, and electrophoresis, generally using the same procedures as have been published for use in purifying the naturally occurring material.

While recombinant production of the analogs is the most convenient and practical method of preparing the proteins, it may also be desirable to synthesize the analogs using protein synthesis techniques, such as standard solid-phase peptide synthesis technology. This approach may be suitable in particular in the case of truncated forms of the RCA protein family having modified amino acid sequences, especially in view of the discovery that proteins containing as few as three SCRs have useful biological activity.

### Assay Systems

The analogs are tested for the desired biological activities among those characteristic of the RCA family using *in vitro* or *in vivo* assays. *In vitro* systems such as those described by Wong and Farrell (J. Immunol. (1991) 146:656) can be used to measure effects on the complement pathways. *In vivo* and general biological effects can be assessed as described by Weisman, et al. (Science (1990) 249:146); or Yeh, et al. (J. Immunol. (1991) 146:250), the teachings of which are incorporated by reference.

### Binding Assays.

Affinity chromatography columns were prepared as described by Krych, et al., Proc. Natl. Acad. Sci. USA 88, 4353-4357. Binding assays were performed using 100 ml of iC3-Sepharose (iC3-S) or C4b-Sepharose (C4b-S) and 1.8 ml of medium containing of the mutant protein. Media were diluted to desired concentrations of NaCl. After 1 hr on a rotator at room temperature, samples were centrifuged, media removed and bound protein eluted from the Sepharose using 400 mM NaCl with 1% NP-40. Eluted proteins were quantitated by ELISA, using two monoclonal anti-CR1 antibodies, 3D9 and E11 (Hogg, et al., Eur. J. Immunol. 14, 236-240 (1984), O'Shea, et al., J. Immunol. 134, 2580-2587 (1985)). Since neither monoclonal antibody reacts with CR1 SCRs-1,2,8 or 9, all of the mutants derived from either CR1-4 or CR1-4(8,9) could be quantitated using this assay. For each mutant protein at least three binding assays from different transfections were performed.

### Assay for Cofactor Activity

C3 and C4 were purified according to the method of Dykman, et al., Proc. Natl. Acad. Sci. USA 80, 1698-1702 (1983), Dykman, et al., J. Exp. Med. 157, 2160-2165 (1983) or purchased (Quidel, San Diego, CA), converted to C3b and C4b and labelled with ¹²⁵I using Iodogen coated beads (Pierce). Cofactor assays were performed using 200 ng of labelled C3b or C4b, 60 ng of factor I (Quidel) and media with mutant proteins. Amounts of the cofactor proteins were estimated in ELISA assay based on a standard curve of secreted CR1 (sCR1, Weisman, et al., Science 249, 146-151 (1990)). To test for cleavage of C3b, samples containing approximately 6 pg of the mutant proteins were incubated for 1 hr at 37°C. To test for cleavage of C4b, samples were incubated for up to 16 hrs at 37°C. After incubation, samples were reduced by boiling in the buffer containing 2% SDS and 5% beta-mercaptoethanol in 0.25% TRIS, pH 6.8 and electrophoresed on a 4-20% SDS-PAGE (Integrated Separations) or on a 10% self-made gel. After drying the gels were autoradiographed at -70°C using an intensifying screen.

### Preparation and Administration of Pharmaceutical Compositions

The most potent analogs based on the in vitro assays are tested *in vivo*. In general, the *in vitro* assays are accepted as highly correlated with the corresponding *in vivo* activity. The appropriate dosages are determined by comparing the *in vitro* activity of the naturally occurring protein with that of the analog, comparing the *in vitro* activity of the naturally occurring protein with the *in vivo* activity of the naturally occurring protein, then calculating the expected *in* *vivo* activity of the analog, adjusting for any measured differences in half-life.

Complement activation can account for substantial tissue damage in a wide variety of autoimmune/immune complex mediated syndromes such as systemic lupus erythematosus, rheumatoid arthritis, hemolytic anemias, myasthenia gravis and others. Inhibition of the complement system is a desirable therapeutic intervention in these cases. In some instances, specific inhibition of the classical pathway alone by RCA analogs could be preferred since long-term inhibition of the alternative pathway could lead to side effects.

Inhibition of complement activation could also be desirable in cases that involve tissue damage brought about by vascular injury such as myocardial infarction, cerebral vascular accidents or acute shock lung syndrome. In these cases, the complement system may contribute to the destruction of partially damaged tissue as in reperfusion injury. Highly stringent inhibition of complement for relatively brief periods might be preferred in these instances and soluble RCA analogs designed for higher potency may prove especially useful.

Complement inhibition may also prove important in the prevention of xenograft rejection. Organs derived from animals transgenic for human DAF or MCP may be protected at least in part from complement-mediated hyperacute rejection by the expression of transgenic DAF or MCP on the cell surfaces of the xenograft. Animals transgenic for RCA analogs designed for higher potency may provide more successful xenografts. Soluble RCA analogs may also prove useful in protecting the transplant in the recipient.

Soluble analogs having decreased activity may also be useful as competitive inhibitors of the natural inhibitors, in cases where an increased complement mediated response is desirable or where an individual has a disorder in which their immunity is compromised by overproduction of the natural inhibitors.

The analogs can be administered locally or systemically in pharmaceutically acceptable carries such as saline, phosphate buffered saline, or a controlled release formulation. The dosage level and mode of administration of the analogs depend on the nature of the analog, the nature of the condition to be treated, and the history of the individual patient. Systemic administration is generally required, which may be by injection or by transmucosal or transdermal delivery. Administration by injection may be intravenous, intramuscular, intraperitoneal or subcutaneous. Formulations for injection are generally biocompatible solutions of the active ingredient such as Hank's solution or Ringer's solution. Formulations for transdermal or transmucosal administration generally include penetrants such as fusidic acid or bile salts in combination with detergents or surface-active agents. The formulations can then be manufactured as aerosols, suppositories, or patches. Oral administration is generally not favored for protein or peptide active ingredients; however, if suitably formulated so as to be protected from the digestive enzymes, oral administration can also be employed.

Suitable formulations for a desired mode of administration can be found in Remington's Pharmaceutical Sciences, latest edition, Mack Publishing Co., Easton, PA. The dosage levels and precise formulations are obtainable by routine optimization procedures as is generally known in the art.

### Diagnostic Applications

The analogs which are capable of binding C3b and/or C4b are useful as diagnostic tools in assessing the presence, absence or amount of C3b or C4b or C3b/C4b-bearing immune complexes in biological fluids. Such assays take advantage of the ability of the analog specifically to bind C3b and/or C4b and can be conducted in a variety of formats as is generally known. Formats for specific binding partner assays include direct and competitive formats, sandwich assays, and agglutination assays. Complexation between members of the specific binding pair can be conducted in solution or on a solid phase and can be detected using a variety of labeling techniques including fluorescence, radioisotopes, chromophores, and visible particles.

Typical reagent kits useful in assays for C3b and/or C4b and/or C3b/C4b-bearing immune complexes include the analog specifically binding to the analyte, optionally coupled to a solid support and additional labeling reagents useful in the assay. For example, one of many formats for the assay might include treating the sample to be tested with a solid support to which is coupled the analog as a specific binding partner, washing the support which has been treated with sample suspected of containing analyte, and then treating the washed support with anti-C3b or anti-C4b antibody labeled with an enzyme such as horseradish peroxidase. The presence of labeled enzyme on a support then is detected by addition of a substrate solution which results in the development of a color in the presence of the enzyme.

The present invention will be further understood by reference to the following nonlimiting examples.

### Example 1: Binding Specificity of Truncated CR1.

The cDNA which encodes the first 543 amino acids of mature CR1 (SCR 1-8 and 1/2 of SCR-9) was transfected into COS cells to obtain a secreted protein designated CR1-4.

Two mouse monoclonal anti-CR1 antibodies were used to determine the immunoreactivity of CR1-4, and as a method to assay for this protein. Antibody E11 (Hogg, N., et al., Eur. J. Immunol. (1984) 14:236-243), and 3D9 (O'Shea, J.J., et al., J. Immunol. (1985) 134:2580-2587), recognized CR1 and bound to the recombinantly produced CR1-4.

Binding to C4b or to C3b was tested using either C4b or iC3 (C3 containing a broken thioester bond analogous in reactivity and function to C3b) bound to a Sepharose ^{TM} support as described by Dykman, T., et al., Proc. Natl. Acad. Sci. USA (1983) 80:1698-1702, and Cole, J.L., et al., Proc. Natl. Acad. Sci. USA (1985) 82:859-863. Binding to these solid-supported substrates was verified by testing with an ELISA for CR1.

The C4b derivatized support was able to bind CR1-4. Most efficient binding occurred at between 12.5 and 25 mM salt.

Solubilized C4b inhibited binding of the CR1-4 protein to C4b-derivatized support but soluble iC3 did not inhibit this binding. This confirms that CR1-4 binds primarily C4b but not iC3 (C3b).

### Example 2: Construction of CR1-4 Analogs.

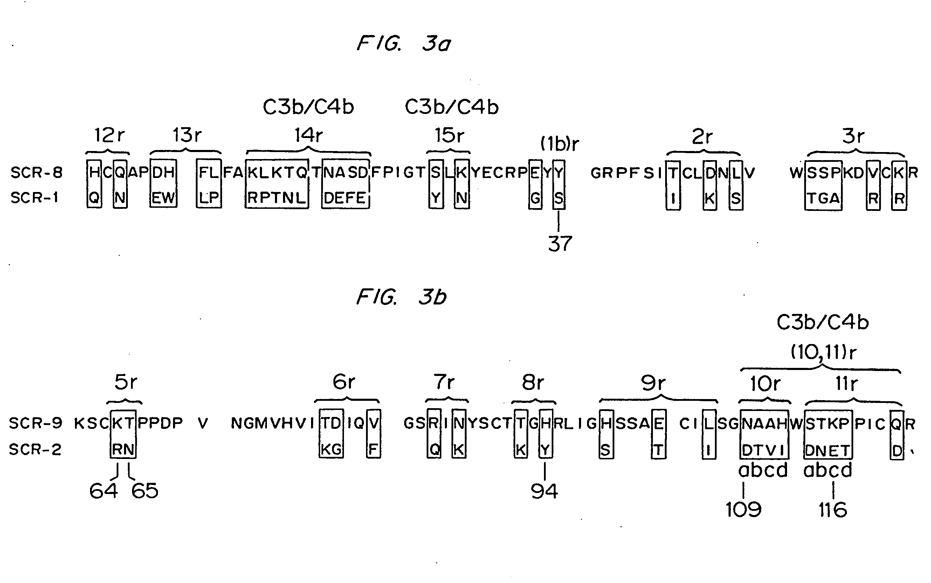

Various mutated forms of CR1-4 were constructed and tested for binding activity to C4b and iC3 as described above in Example 1. Table 2 shows the analogs constructed and the binding and cofactor activities of the analogs. In Table 2, the modifications are shown by the number of the amino acid and the conversion effected. The numbers correspond to those set forth in Figures 3A and 3B which set forth the amino sequences of SCR-1 (Sequence ID No. 1), SCR-2 (Sequence ID No. 3), SCR-8 (Sequence ID No. 2) and SCR-9 (Sequence ID No. 4) in CR1.

The combined changes shown in truncated forms should be predictive of the activity of a modified full length protein. There is strong evidence available that establishes three primary binding and cofactor sites in the major polymorphic form (30 SCRs) of CR1. One site interacts almost exclusively with C4b (SCRs 1-4) while the other two sites, nearly identical in amino acid sequence, interact with both C4b and C3b (SCRs 8-11 and SCRs 15-18) [Klickstein, L.B., et al., J. Exp. Med. 168: 1699-1717 (1988); Kalli, et al., J. Exp. Med. 174: 1451-1460 (1991); Makrides, S.C., et al., J. Biol. Chem. 267: 24754-24761 (1992)].

Electron microscope analysis of CR1 suggests that CR1 is a semi-rigid rod composed of individual globular domains (the SCRs). Thus, CR1 is pictured as an elongated protein bearing well-separated active sites. These sites may not work cooperatively. A comparison of several polymorphic forms, containing the first site (SCRs 1-4) and one, two and three copies of the second site, demonstrated little difference among them in cofactor and decay acceleration assays, as reported by Seya, T., et al., J. Immunology 135: 2661 (1985). Indeed, after the filing of the application to which this claims priority, it was shown that modified membrane forms of CR1 bearing a single active site alone (SCR 1-4 or SCR 15-18) could protect CHO cells from human complement-mediated lysis (Makrides, S.C., et al., J. Biol. Chem. 267: 24754-24761 (1992)). This provides support evidence that a simple CR1 form with a single active site could be biologically active, and that modifications that improve the activity of each primary site in the constructs could be incorporated together in a multiple-site CR1 form, yielding an improved regulator.

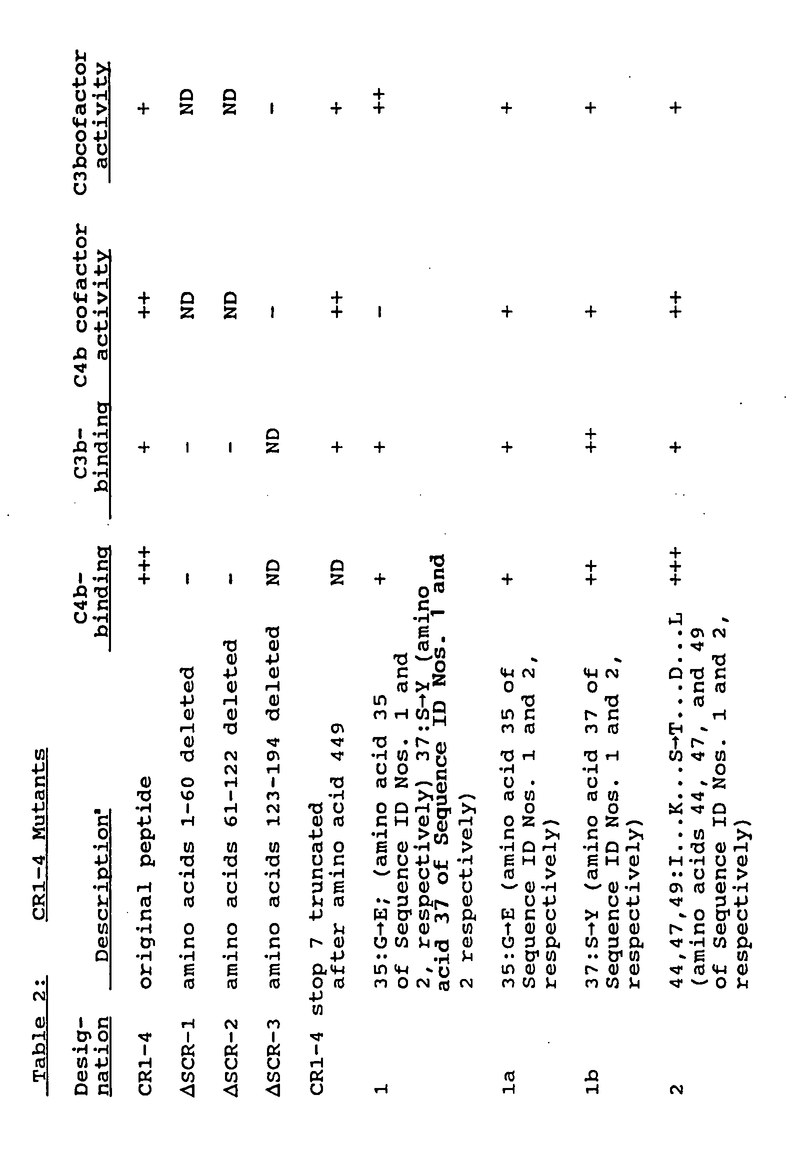

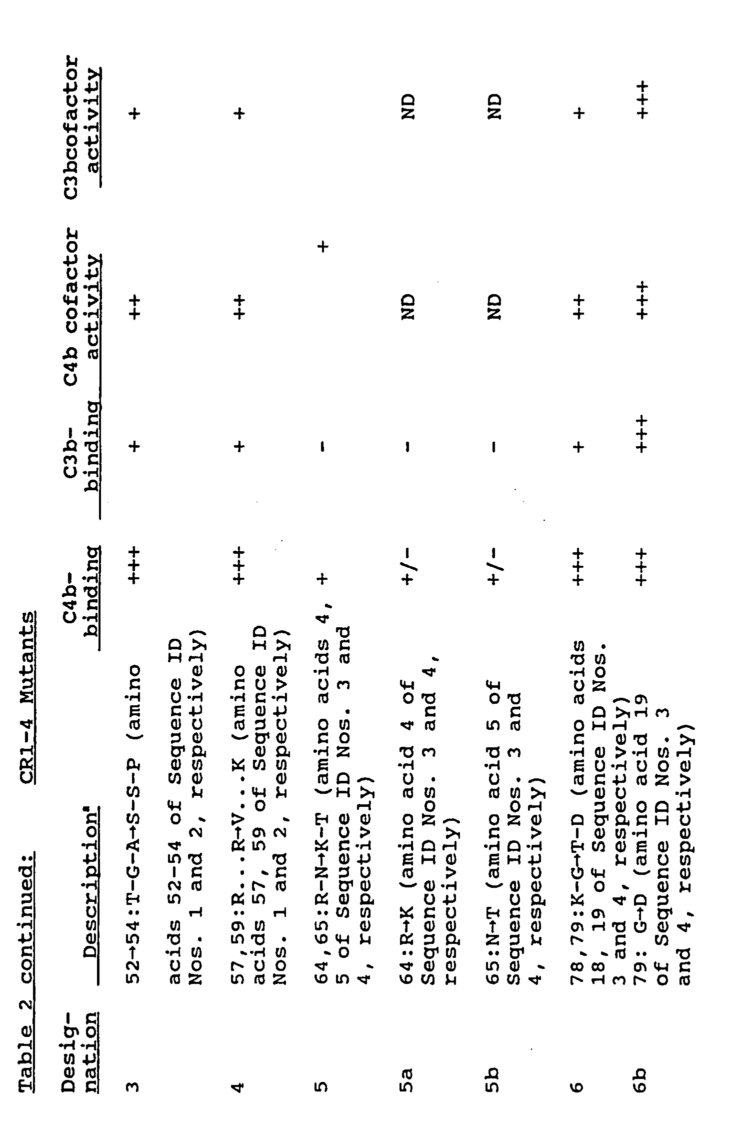

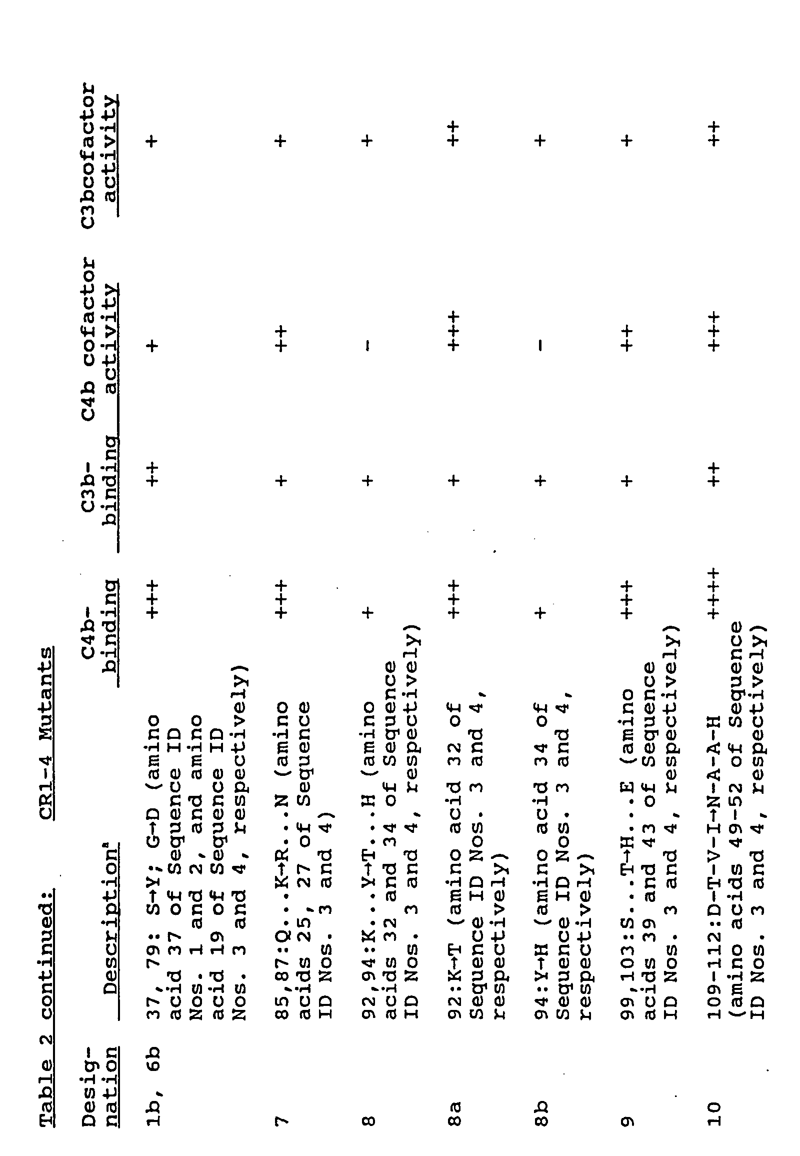

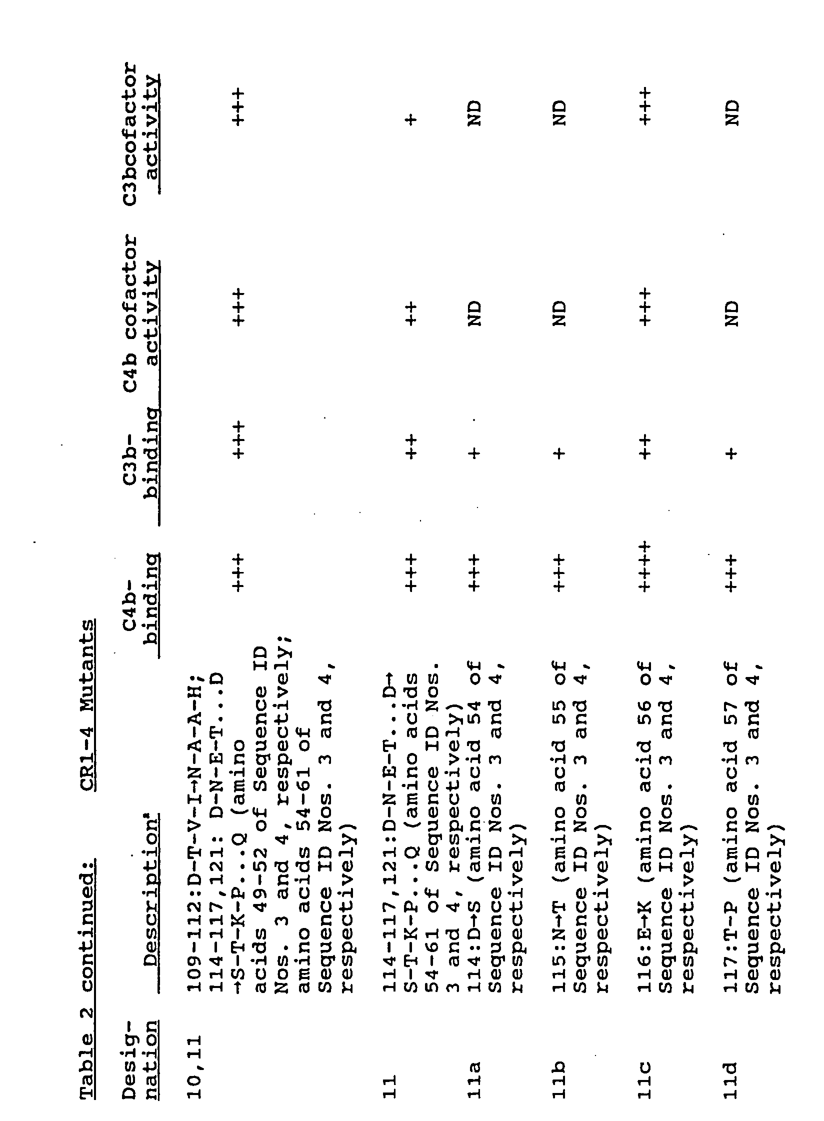

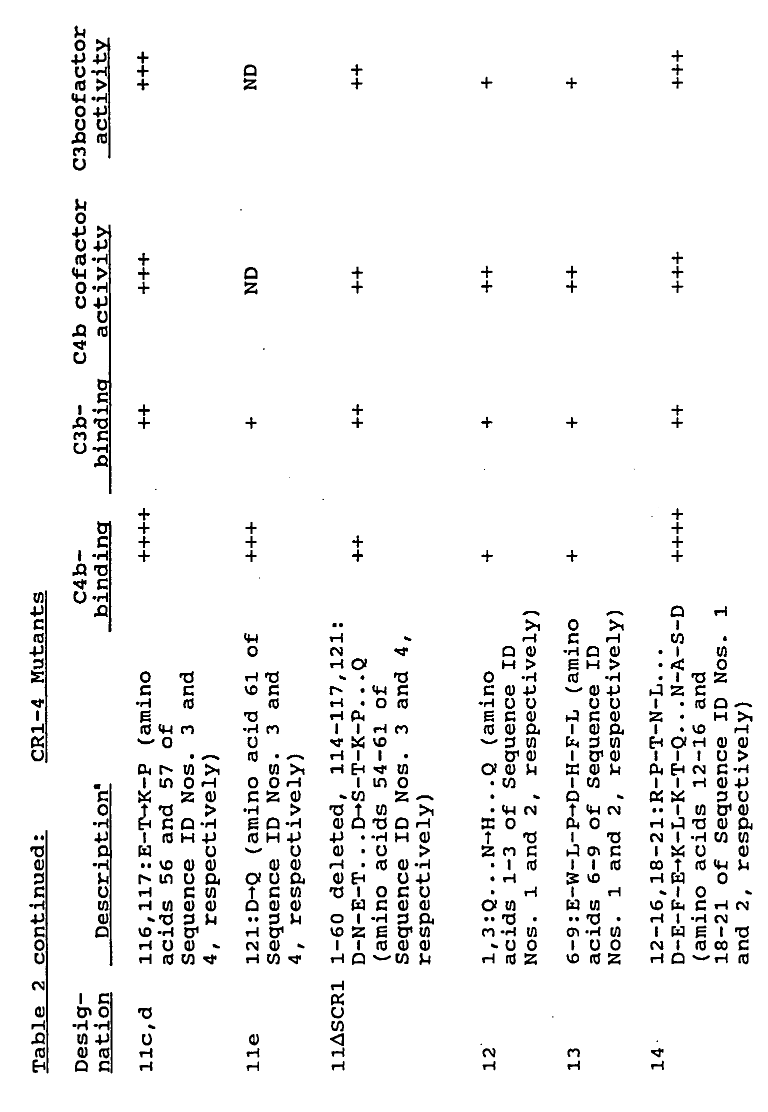

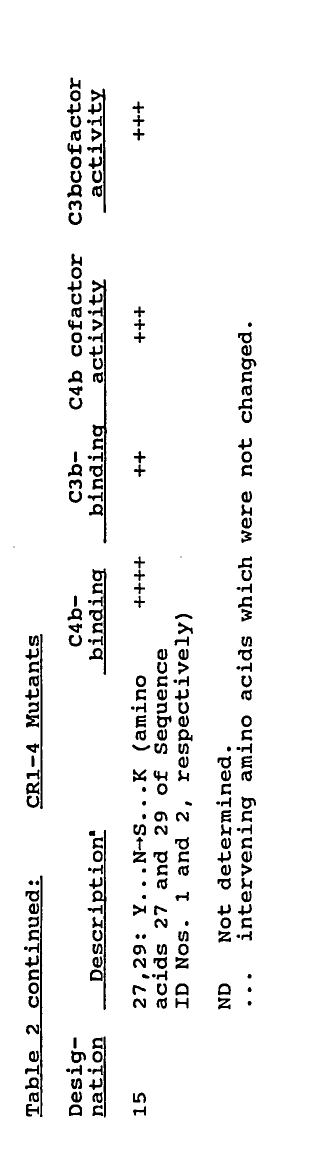

As shown in Table 2, deletion of either SCR-1 or SCR-2 results in loss of C4b-binding activity; thus both regions are required for binding to C4b. Binding to C3b is conferred by insertion of the SCR-9 sequence S-T-K-P-(P-I-C)-Q (amino acids 54-61 of Sequence ID No. 4) into SCR-2 (Sequence ID No. 3); however, deletion of SCR-1 (Sequence ID No. 1) partially eliminates binding to C3b in this analog. Thus, efficient binding to C3b requires not only the relevant sequence in SCR-2 (Sequence ID No. 3), but an additional portion of SCR-1 (Sequence ID No. 1). The ability to bind C3b conferred by altering the sequence in SCR-2 (Sequence ID No. 3) does not affect binding to C4b.

As also shown by Table 2, it is possible to weaken or destroy binding to C4b by altering amino acids 35, 64-65 or 94. It is possible to strengthen C4b binding by altering amino acid 92.

These results indicate that by manipulation of C3b and C4b binding sites in CR1 the affinity and specificity of CR1-4 can be altered. Similar alterations may be made to corresponding regions in additional members of the RCA protein family.

### Example 3: Construction of a More Potent Soluble CR1 Analog.

A stringent inhibitor of the complement system has applications where higher potency is required. In this example, the sCR1 sequence is used as starting material for a family of analogs of higher inhibitory capacity for both classical and alternative pathways. Other CR1 truncated, full-length, recombined or hybrid forms or CR1 could also be used.

As noted above, the sCR1 protein contains four corresponding regions: SCRs 1-2 (Sequence ID Nos. 1 and 3, respectively), SCRs 8-9, (Sequence ID Nos. 2 and 4, respectively) SCRs 15-16 and SCRs 22-23. The primary C4b-binding site is within SCRs 1-2 (Sequence ID Nos. 1 and 3, respectively) and the two primary C3b-binding sites are in SCRs 8-9 (Sequence ID Nos. 2 and 4, respectively) and SCRs 15-16. SCRs 22-23 has no reported binding activity although it is highly homologous in amino acid sequence to the other three corresponding regions.

One or more substitutions are introduced into the corresponding positions of soluble CR1 SCRs 1-2, 8-9, 15-16, and 22-23. Specific substitutions designed to increase C3b and C4b cofactor activity and binding activity are selected from the following:

| positions | substitution |
|---|---|
| 35 | G (amino acid 35 of Sequence ID No. 1) |
| | |
| 64-65 | R-N (amino acids 4-5 of Sequence ID No. 3) |
| | |
| 94 | Y (amino acid 34 of Sequence ID No. 3) |
| | |
| 109-112 | N-A-A-H (amino acids 49-52 of Sequence ID No. 4) |
| | |
| 114-121 | S-T-K-P-P-I-C-Q (amino acids 54-61 of Sequence ID No. 4) |
| | |
| 109-121 | N-A-A-H-W-S-T-K-P-P-I-C-Q (amino acids 49-61 of Sequence ID No. 4) |
| | |
| 92 | T (amino acid 32 of Sequence ID No. 4) |
| | |
| 79 | D (amino acid 19 of Sequence ID No. 4) |
| | |
| 12-16 | K-L-K-T-Q (amino acids 12-16 of Sequence ID No. 2) |
| | |
| 18-21 | N-A-S-D (amino acids 18-21 of Sequence ID No. 2) |
| | |
| 12-21 | K-L-K-T-Q-T-N-A-S-D (amino acids 12-21 of Sequence ID No. 2) |
| | |
| 27-29 | S-L-K (amino acids 27-29 of Sequence ID No. 2) |

In some cases, some of these amino acids may already be present in the corresponding position. In some cases structurally similar amino acids may be substituted instead of those substituted above.

The use of these substitutions at some or all four corresponding positions will yield a full length soluble CR1 form with more potent active sites, and with an additional active site at SCR 22-24.

Substitutions described above were introduced into the four corresponding regions of interest in order to increase the affinity of sCR1 for its C3b and C4b-containing targets. These modifications were designed to increase the use of existing coenzyme and dissociation functions and potentially to establish new coenzyme and dissociation functions. The introduction of amino acid sequences significant to C3b binding into the C4b binding region presumably would not interfere substantially with C4b activities since such a substitution in CR1-4 did not detectably alter the C4b binding properties of CR1-4. The introduction of amino acids significant to C4b-binding into C3b-binding regions presumably would not interfere substantially with C3b binding activities since substantial C3b-binding occurs in CR1-4 mutant 11, in which many amino acids specific to SCRs 1-2, including those significant in C4b binding, are already present.

Specific substitutions designed to increase affinity to C4b were described in Example 3: SCRs 1-2 (Sequence ID Nos. 1 and 3), the primary C4b- binding site of CR1, modified by replacement of I (amino acid 32 of Sequence ID Nos. 3) at position 92 with T (amino acid 32 of Sequence ID No. 4); SCRs 8-9 (Sequence ID Nos. 2 and 4) and the identical SCRs 15-16 modified by replacement of E (amino acid 35 of Sequence ID No. 2) at a position corresponding to 35 by G (amino acid 35 of Sequence ID No. 1) (or A), H (amino acid 34 of Sequence ID No. 4) at a position corresponding to 94 by Y (amino acid 34 of Sequence ID No. 3 (or F), K (amino acid 4 of Sequence ID No. 4) at a position corresponding to 64 by R (amino acid 4 of Sequence ID No. 3) and T (amino acid 5 of Sequence ID No. 4) at a position corresponding to 65 by N (amino acid 5 of Sequence ID No. 3) (or Q). SCRs 22-23 were also modified by replacement of G at a position corresponding to 64 by R (amino acid 4 of Sequence ID No. 3) (or K ((amino acid 4 of Sequence ID No. 4)), P at a position corresponding to 65 with N (amino acid 5 of Sequence ID No. 3 (or Q), E at a position corresponding to 92 with T (amino acid 32 of Sequence ID No. 4) and F at a position corresponding to 94 with Y amino acid 34 of Sequence ID No. 3).

Together, these modifications result in higher affinity for C4b and may also result in the establishment of new coenzyme or dissociation functions or the improvement in the capacity of the coenzyme and dissociation functions present on the sCR1 starting material.

As described in previous examples, substitution of a short stretch of amino acids into SCRs 1-2 (Sequence ID Nos. 1 and 3) of CR1-4 by corresponding amino acids of SCRs 8-9 (Sequence ID Nos. 2 and 4) confers C3b binding capacity to CR1-4. Replacement of D-N-E-T-P-I-C-D (amino acids 54-61 of Sequence ID No. 3) at position 114-121 in SCR1 SCRs 1-2 by S-T-K-P-P-I-C-Q (amino acids 54-61 of Sequence ID No. 4) increases the affinity of SCR1 for C3b. Further, replacement of D-K-K-A-P-I-C-D (Sequence ID No. 5) at positions 114-121 in SCRs 22-23 by S-T-K-P-P-I-C-Q (amino acids 54-61 of Sequence ID No. 4) increases the affinity of sCR1 for C3b. Some structurally similar amino acids may also be substituted in the S-T-K-P-P-I-C-Q (amino acids 54-61 of Sequence ID No. 4) sequence.

A more potent complement inhibitor, in general, provides increased C4b-binding and increased C3b-binding. This is achieved by introducing all the modifications set forth above.

### Example 4: Construction of Truncated CR1 mutants having increased activity.

More than 25 mutants were assayed for cofactor activity at several different salt concentrations. The assay was done as described in Adams, E.M., Brown, M.C., Nunge, M., Krych, M., and Atkinson, J.P. (1991) J. Immunology, 147: 3005-3011.

A number of mutations of the active sites in SCRs 1-3, the site that normally interacts with C4b and not C3b, which improve on the natural activity of this site, both for C4b and C3b cofactor activity. At least one mutant in the active sites in SCR 8-11, which is apparently the more potent natural active site, that improves on the cofactor activity of this site for both C3b and C4b was also detected.

In summary, specific CR1 proteins that have both C3b and C4b cofactor activity and are in some ways an improvement on the full length soluble CR1 have now been identified. These constructs are less than one-fourth (seven SCR constructs) to a third the size of soluble CR1 (sCR1) and have both C4b binding and cofactor activity as well as C3b binding and cofactor activity. Constructs including as few as three SCRs appear to have C3b cofactor activity, indicative of C3b binding activity. In some cases the data indicates that the modified active site appears to be more potent than the natural active sites.

These forms and their activities are shown in Table 3:

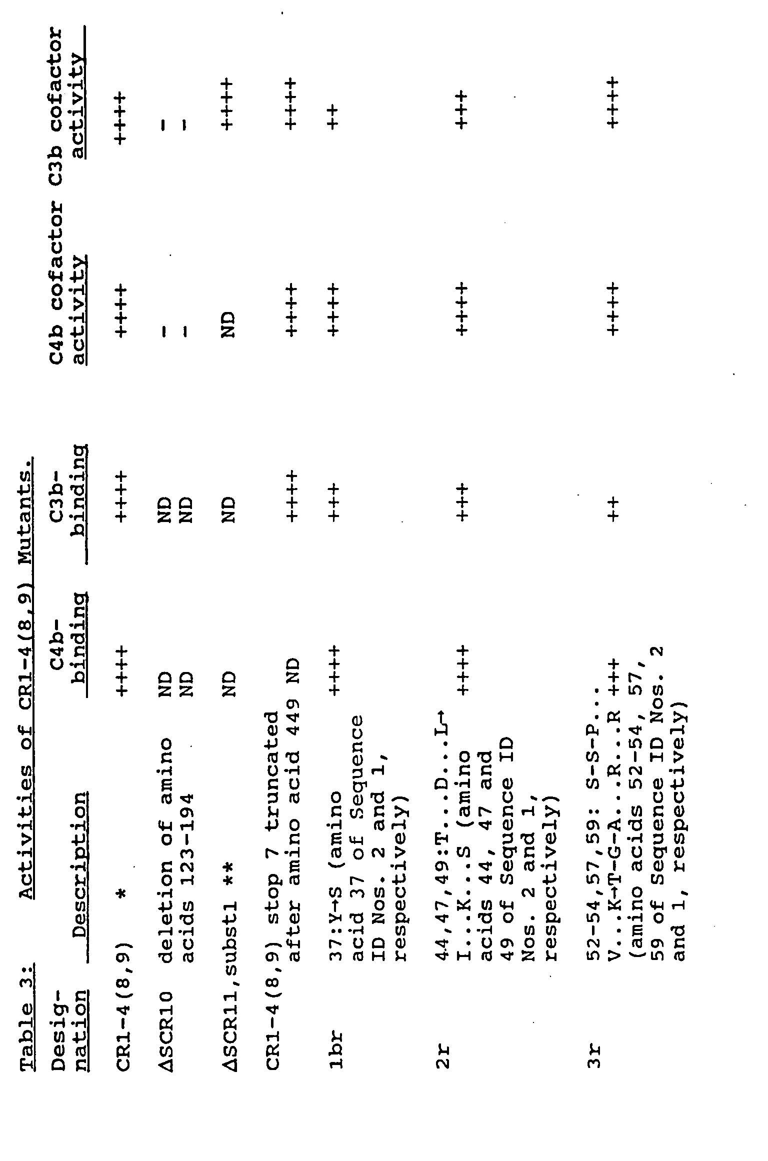

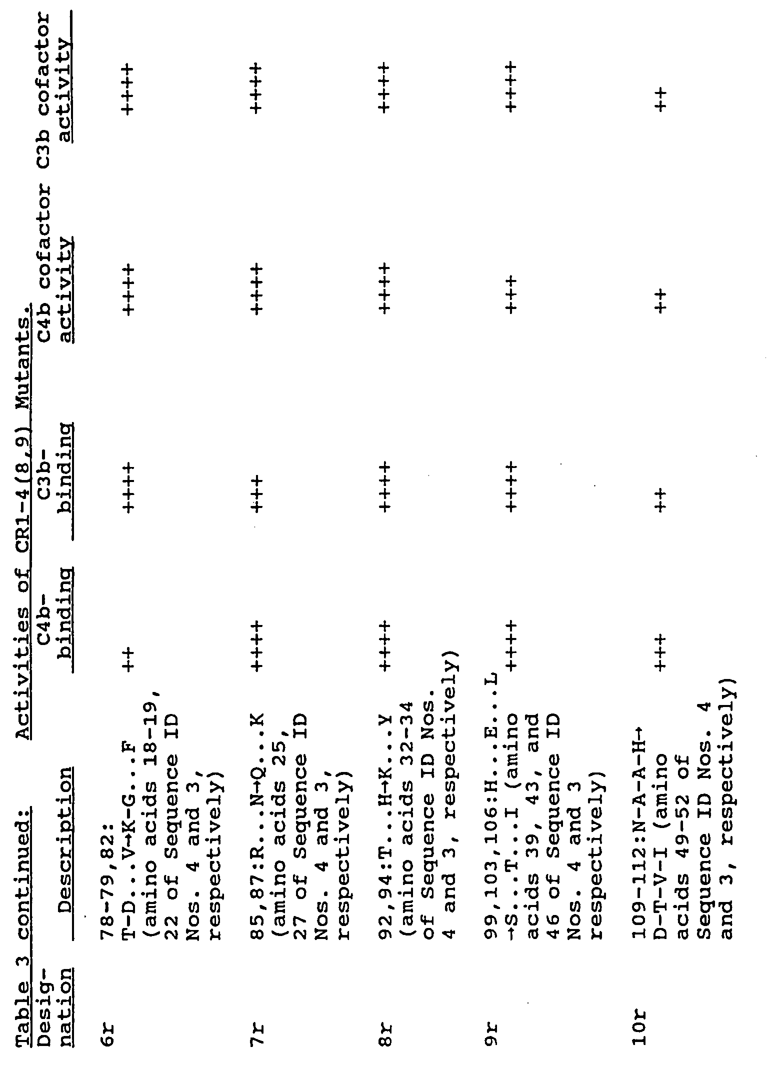

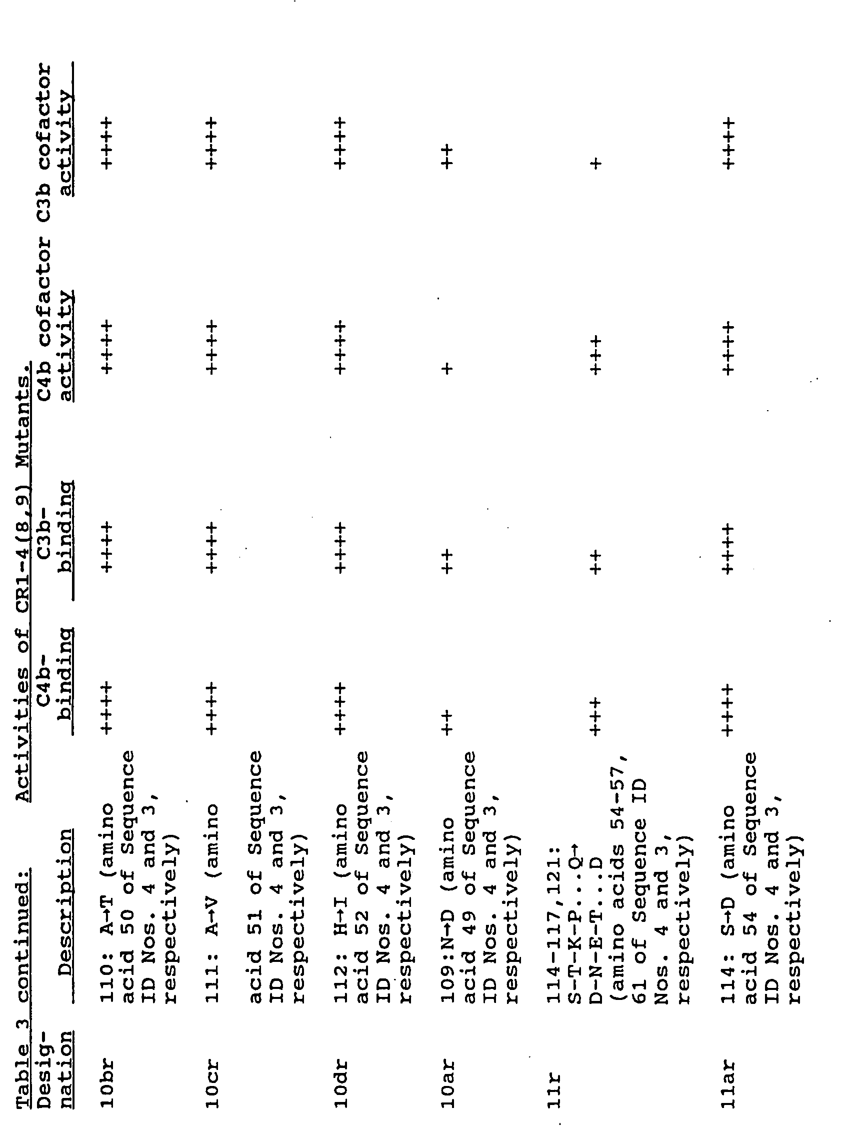

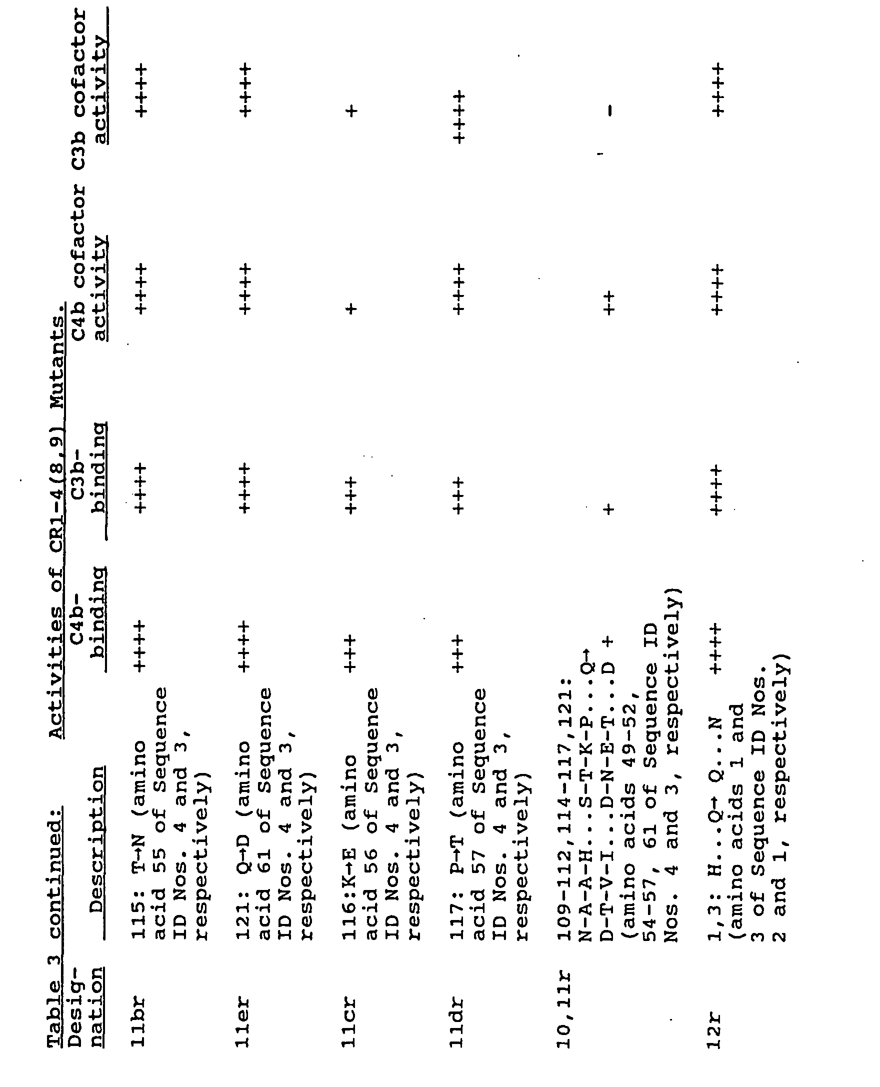

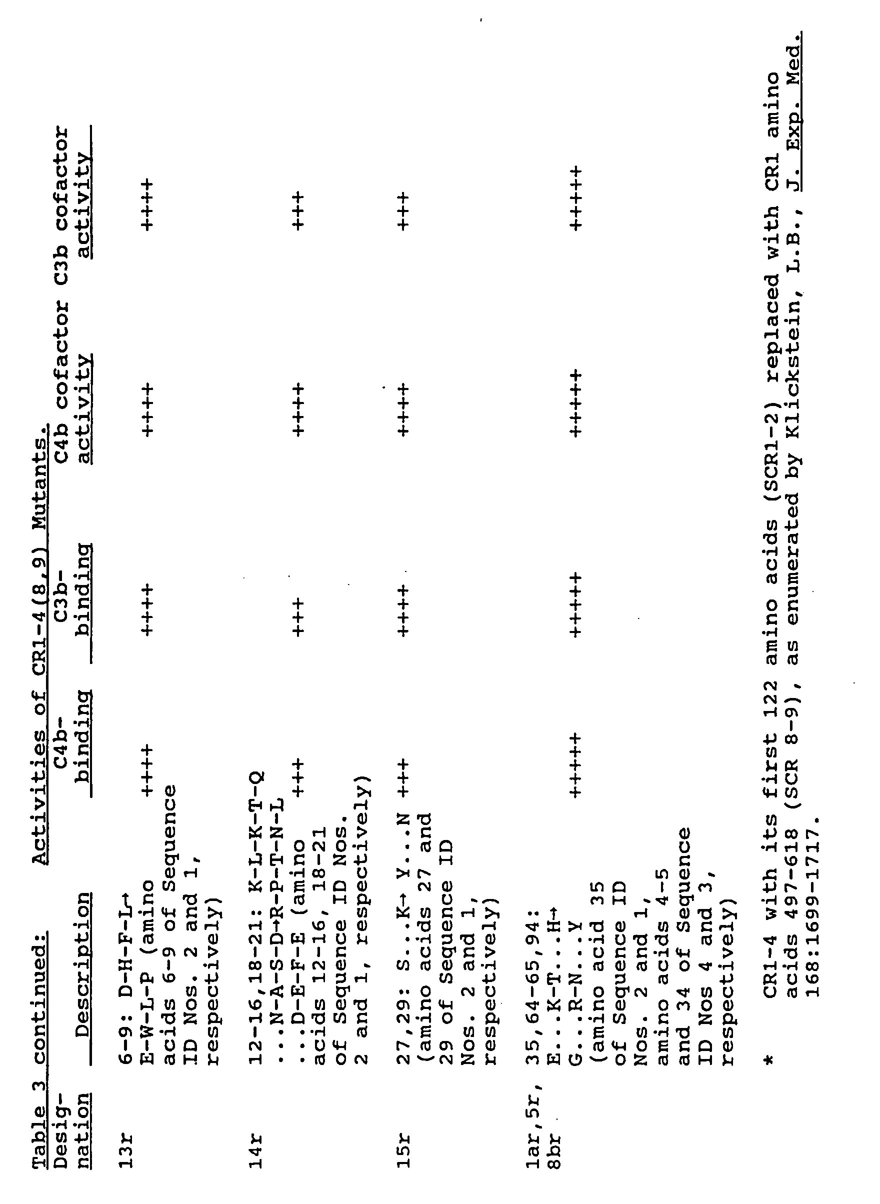

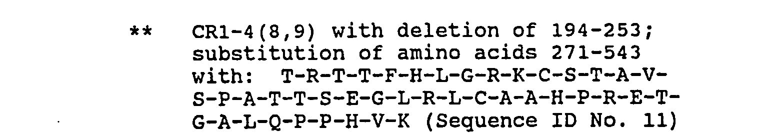

### Example 5: DAF Analogs.

The membrane-bound complement regulator DAF facilitates the dissociation of C3b and C4b-containing convertases but does not bind C3b or C4b, nor does it serve as cofactor for factor 1-mediated proteolytic inactivation of C3b or C4b. It would be desirable, under certain situations, to increase the complement regulatory activity of DAF or of truncated, recombined or hybrid forms of DAF.

Based on amino sequence homology, DAF SCRs 2-3 corresponds to the CR1 active regions. Homology between DAF SCRs 2-3 and CR1 SCRs 1-2 is 40% while homology between DAF SCRs 2-3 and CR1 SCRs 8-9 is 39%. Since there are several unmatched amino acids in these alignments, the position numbers of DAF do not precisely match those of CR1.

One or more substitutions are introduced into the DAF SCRs 2-3. Specific substitutions designed to confer C3b and C4b cofactor activity and binding activity are selected from the following:

DAF position(s) enumerated as in Lublin and Atkinson, Ann. Rev. Immunol. 9:431 (1991):

| DAF position | DAF sequence | Substitution |
|---|---|---|
| 180-187 | S-D-P-L-P-E-C-R (Sequence ID No. 6) | S-T-K-P-P-I-C-Q (AA 54-61 of Seq. ID No. 4) |
| | | |
| 175-178 | S-S-V-Q (Sequence ID No. 7) | N-A-A-H (amino acids 49-52 of Sequence ID No. 4) |
| | | |
| 175-187 | S-D-P-L-P-E-C-R-S-S-V-Q (Sequence ID No. 8) | S-T-K-P-P-I-C-Q-N-A-A-H (Sequence ID No. 9) |
| | | |
| 130 | P | R |
| | | |
| 145 | G | D |
| | | |
| 77-84 | Q-P-Y-I-T-Q-N-Y (Sequence ID No. 10) | K-L-K-T-Q-T-N-A-S-D (amino acids 12-21 of Sequence ID No. 2) |
| | | |
| 90-92 | V-V-E | S-L-K (amino acids 27-29 of Sequence ID No. 2) |

Replacement of S-D-P-L-P-E-C-R (Sequence ID No. 6) at DAF position 180-187 (using the enumeration in Lublin and Atkinson, Ann. Rev. Immunol. (1989) 7:35-58) with S-T-K-P-P-I-C-Q (AA 54-61 of Seq. ID No. 4) increases the affinity of DAF for C3b. Replacement of S-D-P-L-P-E-C (amino acids 1-7 of Sequence ID No. 6) with S-T-K-P-P-I-C-Q (amino acids 54-61 of Sequence ID No. 4) leaves R at the end of this segment, since R is found in the corresponding positions of CR1 SCR 1-2 (Sequence ID Nos. 1 and 3) and CR1 SCR 8-9 (Sequence ID Nos. 2 and 4). Some structurally similar amino acids can be substituted in the S-T-K-P-P-I-C-Q (amino acids 54-61 of Sequence ID No. 4) sequence.

Replacement of P at DAF position 130 with R increases the affinity of DAF for C4b. All other amino acids found important in C4b interactions in CR1 are already present in DAF SCRs 2-3.

These substitutions, by increasing the affinity of DAF for C3b and, possibly, C4b, enhance the respective 5 inhibitory effects of DAF on complement activation.

### Example 6: Analogs of Factor H.

Factor H is a plasma protein consisting solely of 20 SCRs. Factor H exhibits C3b binding and C3b cofactor and dissociation capacity but no apparent ability to inactivate or bind C4b. Since factor H has already evolved as a plasma protein, it could be advantageous to use it as the starting material for soluble RCA analogs. Although the active sites of factor H are not precisely known, a proteolytic fragment composed of the first 5.5 SCRs of factor H exhibits C3b-binding and cofactor activity (Alsenz et al., Biochem. J. (1984) 389).

To increase the affinity of factor H for C3b binding, N-A-A-H-W-S-T-K-P-P-I-C-Q (amino acids 49-61 of Sequence ID No. 4) is introduced into several of the SCRs, none bearing a close correspondence to CR1 SCR 8-9. In one embodiment, the first six SCRs are left unmodified, thus retaining the original active site(s), and the remaining fourteen SCRs are modified by substitution of the N-A-A-H-W-S-T-K-P-P-I-C-Q (amino acids 49-61 of Sequence ID No. 4) into the position(s) homologous to CR1. Some structurally similar amino acids can substitute in the N-A-A-H-W-S-T-K-P-P-I-C-Q (amino acids 49-61 of Sequence ID No. 4) sequence. Homologous positions are readily identified because the W that precedes this C3b binding segment is found in most SCRs: in all factor H SCRs except SCR 10 and SCR 20 while the C within the C3b binding segment is found in all the known SCRS. While not all substitutions will necessarily confer added binding activity, since these factor H SCRs are less homologous to the CR1 SCR 1-2 and CR1 SCR 8-9 regions. However, at least some modified H SCRs gain C3b-binding capacity, resulting in a factor H analog with much higher affinity for C3b. As discussed above, higher affinity would lead to the greater use of the active sites already present in the first six H SCRS.

## Claims

1. An analog of a protein regulating complement activation having short consensus repeats of amino acid sequence selected from the group consisting of complement receptor 1, complement receptor 2, decay accelerating factor, membrane cofactor protein, C4 binding protein, and factor H, and these complement regulating proteins wherein the carboxy terminus is removed to allow the protein to be secreted, wherein said protein analog is selected from the group consisting of complement regulating proteins containing short consensus repeats derived from a second, different complement regulating protein, complement regulating proteins wherein the short consensus repeats are rearranged, complement regulating proteins having defined amino acid substitutions in the short consensus repeats selected from the group consisting of repeats having binding activity, cofactor activity, and decay accelerating activity, wherein the substitution alters the activity of the naturally occurring complement regulatory protein, and complement regulating proteins consisting of as few as three short consenses repeats, wherein the protein has complement regulatory activity.

2. The analog of claim 1 wherein the complement regulatory activity is selected from the group consisting of C3b binding activity, C3b cofactor activity, C4b binding activity, C4b cofactor activity, and decay accelerating activity.

3. The analog of claim 2 wherein the protein is complement receptor one.

4. The analog of claim 2 wherein the protein is decay accelerating factor.

5. The analog of claim 2 wherein the protein is factor H.

6. The analog of claim 2 wherein the C3b binding and cofactor activities of the protein are enhanced by substitutions increasing C4b binding of the protein.

7. The analog of claim 2 wherein the C4b binding and cofactor activities of the protein are enhanced by substitutions increasing C3b binding of the protein.

8. The analog of claim 2 wherein the protein contains a change within a short consensus repeat that corresponds with a change to complement receptor one selected from the group consisting of:
CR1-4 with its first 122 amino acids (SCR1-2) (Sequence ID Nos. 1 and 3) replaced with CR1 amino acids 497-618 (SCR 8-9) (Sequence ID Nos. 2 and 4) and CR1-4(8,9) with deletion of 194-253; substitution of amino acids 271-543 with: T-R-T-T-F-H-L-G-R-K-C-S-T-A-V-S-P-A-T-T-S-E-G-L-R-L-C-A-A-H-P-R-E-T-G-A-L-Q-P-P-H-V-K, (Sequence ID No. 11) or structurally similar amino acids.

9. The analog of claim 2 wherein the protein contains a change within a short consensus repeat that corresponds with a change to complement receptor one selected from the group consisting of: 79: D (amino acid 19 of Sequence ID No. 4); 37,79: Y,D (amino acid 37 of Sequence ID No. 2 and amino acid 19 of Sequence ID No. 4); 92: T (amino acid 32 of Sequence ID No. 4); 109-112: N-A-A-H (amino acids 49-52 of Sequence ID No. 4); 109-112, 114-117, 121: N-A-A-H, S-T-K-P...Q (amino acids 49-52, 54-57, 61 of Sequence ID No. 4); 114-117, 121: S-T-K-P...Q (amino acids 54-61 of Sequence ID No. 4); 116: K (amino acid 56 of Sequence ID No. 4); 116,117: K-P (amino acids 56-57 of Sequence ID No. 4); 92-94: K...Y (amino acids 32-34 of Sequence ID No. 3); 99,103,106: S...T...I (amino acids 39, 43 and 46 of Sequence ID No. 3); 109-112: D-T-V-I (amino acids 49-52 of Sequence ID No. 3); 110: T (amino acid 50 of Sequence ID No. 3); 111: V (amino acid 51 of Sequence ID No. 3); 112: I (amino acid 52 of Sequence ID No. 3); 114: D (amino acid 54 of Sequence ID No. 3); 115: N (amino acid 55 of Sequence ID No. 3); 121: D (amino acid 61 of Sequence ID No. 3); 117: T (amino acid 57 of Sequence ID No. 3); 1,3: Q...N (amino acids 1,3 of Sequence ID No. 1); 6-9: E-W-L-P (amino acids 6-9 of Sequence ID No. 1); 12-16, 18-21: K-L-K-T-Q...N-A-S-D (amino acids 12-21 of Sequence ID No. 2); 27,29: S...K (amino acids 27,29 of Sequence ID No. 2); 37: S (amino acid 37 of Sequence ID No. 1); 44, 47, 49: I...K...S (amino acids 44, 47, 49 of Sequence ID No. 1) ; 52-54, 57, 59: T-G-A...R...R (amino acids 52-54, 57, 59 of Sequence ID No. 1); 78-79, 82: K-G...F (amino acids 18-19, 22 of Sequence ID No. 3); 85, 87: Q...K (amino acids 25, 27 of Sequence ID No. 3); 12-16, 18-21: R-P-T-N-L...D-E-F-E (amino acids 12-21 of Sequence ID No. 1); 27,29: Y...N (amino acids 27, 29 of Sequence ID No. 1); 35, 64-65, 94: G...R-N...Y (amino acid 35 of Sequence ID No. 1, amino acids 4-5, 34 of Sequence ID No. 3), substitutions with structurally similar amino acids, and combinations thereof.

10. The analog of claim 2 wherein the complement regulatory protein is decay accelerating factor wherein one or more substitutions are introduced into the region of the protein corresponding to decay accelerating factor short consensus repeats SCRs 2-3 selected from the group consisting of 180-187: S-T-K-P-P-I-C-Q (amino acids 54-61 of Sequence ID No. 4); 175-178: N-A-A-H (amino acids 49-52 of Sequence ID No. 4); 175-187: S-T-K-P-P-I-C-Q-N-A-A-H (Sequence ID No. 9); 130: R (amino acid 4 of Sequence ID No. 3); 145: D (amino acid 19 of Sequence ID No. 4); 77-84: K-L-K-T-Q-T-N-A-S-D (amino acids 12-21 of Sequence ID No. 2); 90-92: S-L-K (amino acids 27-29 of Sequence ID No. 2), substitutions with structurally similar amino acids, and combinations thereof.

11. The analog of claim 1 wherein the complement regulatory protein is factor H comprising sequences conferring on the protein an activity selected from the group consisting of C3b binding activity, C3b cofactor activity, C4b binding activity, and C4b cofactor activity,
wherein the sequences are derived from a protein selected from the group consisting of complement receptor 1, membrane cofactor protein, C4 binding protein, and factor H,.

12. The analog of claim 1 comprising at least one short consensus repeat derived from a different protein selected from the group consisting of complement receptor 1, complement receptor 2, decay accelerating factor, membrane cofactor protein, C4 binding protein, and factor H.

13. The analog of claim 1 wherein the protein comprises C3b cofactor activity, C4b cofactor activity and decay accelerating activity.

14. The analog of claim 1 wherein the protein consists essentially of three short consensus regions and has two complement regulatory activities.

15. The analog of claim 1 further comprising a pharmaceutically acceptable carrier for administration to a patient in need thereof.

16. A method for making an analog of a protein regulating complement activation having short consensus repeats of amino acid sequence selected from the group consisting of complement receptor 1, complement receptor 2, decay accelerating factor, membrane cofactor protein, C4 binding protein, and factor H, and these complement regulating proteins wherein the carboxy terminus is removed to allow the protein to be secreted,
wherein said protein analog is selected from the group consisting of complement regulating proteins containing short consensus repeats derived from a second, different complement regulating protein, complement regulating proteins wherein the short consensus repeats are rearranged, complement regulating proteins having defined amino acid substitutions in the short consensus repeats selected from the group consisting of repeats having binding activity, cofactor activity, and decay accelerating activity, wherein the substitution alters the activity of the naturally occurring complement regulatory protein, and complement regulating proteins consisting of as few as three short consenses repeats, wherein the protein has complement regulatory activity.

17. The method of claim 16 wherein the complement regulatory activity is selected from the group consisting of C3b binding activity, C3b cofactor activity, C4b binding activity, C4b cofactor activity, and decay accelerating activity.

18. The method of claim 16 wherein the protein is complement receptor one.

19. The method of claim 16 wherein the protein is decay accelerating factor.

20. The method of claim 16 wherein the protein is factor H.

21. The method of claim 17 wherein the C3b binding and cofactor activities of the protein are enhanced by substitutions increasing C4b binding of the protein.

22. The method of claim 17 wherein the C4b binding and cofactor activities of the protein are enhanced by substitutions increasing C3b binding of the protein.

23. The method of claim 17 wherein the protein contains a change within a short consensus repeat that corresponds with a change to complement receptor one selected from the group consisting of:
CR1-4 with its first 122 amino acids (SCR1-2) (Sequence ID Nos. 1 and 3) replaced with CR1 amino acids 497-618 (SCR 8-9) (Sequence ID Nos. 2 and 4) and CR1-4(8,9) with deletion of 194-253; substitution of amino acids 271-543 with: T-R-T-T-F-H-L-G-R-K-C-S-T-A-V-S-P-A-T-T-S-E-G-L-R-L-C-A-A-H-P-R-E-T-G-A-L-Q-P-P-H-V-K (Sequence ID No. 11), or structurally similar amino acids.

24. The method of claim 17 wherein the protein contains a change within a short consensus repeat that corresponds with a change to complement receptor one selected from the group consisting of: 79: D (amino acid 19 of Sequence ID No. 4); 37,79: Y,D (amino acid 37 of Sequence ID No. 2 and amino acid 19 of Sequence ID No. 4); 92: T (amino acid 32 of Sequence ID No. 4); 109-112: N-A-A-H (amino acids 49-52 of Sequence ID No. 4); 109-112, 114-117, 121: N-A-A-H, S-T-K-P...Q (amino acids 49-52, 54-57, 61 of Sequence ID No. 4); 114-117, 121: S-T-K-P...Q (amino acids 54-61 of Sequence ID No. 4); 116: K (amino acid 56 of Sequence ID No 4); 116,117: K-P (amino acids 56-57 of Sequence ID No. 4); 92-94: K...Y (amino acids 32-34 of Sequence ID No. 3); 99,103,106: S...T...I (amino acids 39, 43, 46 of Sequence ID No. 3); 109-112: D-T-V-I (amino acids 49-52 of Sequence ID No. 3); 110: T (amino acid 50 of Sequence ID No. 3); 111: V (amino acid 51 of Sequence ID No. 3); 112: I (amino acid 52 of Sequence ID No. 3); 114: D (amino acid 54 of Sequence ID No. 3); 115: N (amino acid 55 of Sequence ID No. 3); 121: D (amino acid 61 of Sequence ID No. 3); 117: T (amino acid 57 of Sequence ID No. 3); 1,3: Q...N (amino acids 1,3 of Sequence ID No. 1); 6-9: E-W-L-P (amino acids 6-9 of Sequence ID No. 1); 12-16, 18-21: K-L-K-T-Q...N-A-S-D (amino acids 12-21 of Sequence ID No. 2); 27,29: S...K (amino acids 27 and 29 of Sequence ID No. 2); 37: S (amino acid 37 of Sequence ID No. 1); 44, 47, 49: I...K...S (amino acids 44, 47, 49 of Sequence ID No. 1); 52-54, 57, 59: T-G-A...R...R (amino acids 52-54, 57, 59 of Sequence ID No. 1); 78-79, 82: K-G...F (amino acids 18-19, 22 of Sequence ID No. 3); 85, 87: Q...K (amino acids 25, 27 of Sequence ID No. 3); 12-16, 18-21: R-P-T-N-L...D-E-F-E (amino acids 12-21 of Sequence ID No. 1); 27,29: Y...N (amino acids 27, 29 of Sequence ID No. 1); 35, 64-65, 94: G...R-N...Y (amino acid 35 of Sequence ID No. 1, amino acids 4-5, 34 of Sequence ID No. 3), substitutions with structurally similar amino acids, and combinations thereof.

25. The method of claim 17 wherein the complement regulatory protein is decay accelerating factor wherein one or more substitutions are introduced into the region of the protein corresponding to decay accelerating factor short consensus repeats SCRs 2-3 selected from the group consisting of 180-187: S-T-K-P-P-I-C-Q (amino acids 54-61 of Sequence ID No. 4); 175-178: N-A-A-H (amino acids 49-52 of Sequence ID No. 4); 175-187: S-T-K-P-P-I-C-Q-N-A-A-H (Sequence ID No. 9); 130: R (amino acid 4 of Sequence ID No. 3); 145: D (amino acid 19 of Sequence ID No. 4); 77-84: K-L-K-T-Q-T-N-A-S-D (amino acids 12-21 of Sequence ID No. 2); 90-92: S-L-K (amino acids 27-29 of Sequence ID No. 2), substitutions with structurally similar amino acids, and combinations thereof.

26. The method of claim 16 wherein the complement regulatory protein is factor H comprising sequences conferring on the protein an activity selected from the group consisting of C3b binding activity, C3b cofactor activity, C4b binding activity, and C4b cofactor activity,
wherein the sequences are derived from a protein selected from the group consisting of complement receptor 1, membrane cofactor protein, C4 binding protein, and factor H.

27. The method of claim 16 comprising at least one short consensus repeat derived from a different protein selected from the group consisting of complement receptor 1, complement receptor 2, decay accelerating factor, membrane cofactor protein, C4 binding protein, and factor H.

28. The method of claim 16 wherein the protein comprises C3b cofactor activity, C4b cofactor activity and decay accelerating activity.

29. The method of claim 16 wherein the protein consists essentially of three short consensus regions and has two complement regulatory activities.

30. The method of claim 16 further comprising mixing with the analog a pharmaceutically acceptable carrier for administration to a patient in need thereof.

31. A DNA sequence which encodes the analogs of claim 1.

32. The DNA sequence of claim 31 in an expression system which is capable, when transformed into a compatible recombinant host cell, of expressing a DNA encoding the analog of claim 1; the expression system comprising a DNA encoding the analog operably linked to control sequences compatible with the host.

33. The DNA sequence of claim 31 stably incorporated into the genome of a transgenic animal.

34. A method for enhancing the C4b or C3b cofactor activity of a complement regulatory protein, wherein the protein has-either C3b or C4b cofactor, comprising adding sequences to the protein conferring binding of the other ligand, either C4b or C3b.


SEQUENCE LISTING

(1) GENERAL INFORMATION:

(i) APPLICANT: Washington University
(ii) TITLE OF INVENTION: MODIFIED TRUNCATED COMPLEMENT SYSTEM
REGULATORS
(iii) NUMBER OF SEQUENCES: 11
(iv) CORRESPONDENCE ADDRESS:
(A) ADDRESSEE: Patrea L. Pabst
(B) STREET: 1100 Peachtree Street, Suite 2800
(C) CITY: Atlanta
(D) STATE: Georgia
(E) COUNTRY: USA
(F) ZIP: 30309-4530
(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25
(vi) CURRENT APPLICATION DATA:
(A) APPLICATION NUMBER: US 08/126,505
(B) FILING DATE: 24-SEP-1993
(C) CLASSIFICATION:
(vii) PRIOR APPLICATION DATA:
(A) APPLICATION NUMBER: US 07/695,514
(B) FILING DATE: 03-MAY-1991
(viii) ATTORNEY/AGENT INFORMATION:
(A) NAME: Pabst, Patrea L.
(B) REGISTRATION NUMBER: 31,284
(C) REFERENCE/DOCKET NUMBER: WU101CIP
(ix) TELECOMMUNICATION INFORMATION:
(A) TELEPHONE: (404)-815-6508
(B) TELEFAX: (404)-815-6555

(2) INFORMATION FOR SEQ ID NO:1:

(i) SEQUENCE CHARACTERISTICS:
(A) LENGTH: 60 amino acids
(B) TYPE: amino acid
(C) STRANDEDNESS: single
(D) TOPOLOGY: linear
(ii) MOLECULE TYPE: protein
(iii) HYPOTHETICAL: NO
(iv) ANTI-SENSE: NO
(v) FRAGMENT TYPE: N-terminal
(x) PUBLICATION INFORMATION:
(A) AUTHORS: Hourcade, D. et al.,
(C) JOURNAL: J. Exp. Med.
(D) VOLUME: 168
(F) PAGES: 1255-1270
(G) DATE: 1988
(K) RELEVANT RESIDUES IN SEQ ID NO:1: FROM 1 TO 60
(xi) SEQUENCE DESCRIPTION: SEQ ID NO:1:

Gln Cys Asn Ala Pro Glu Trp Leu Pro Phe Ala Arg Pro Thr Asn Leu
1               5                   10                  15

Thr Asp Glu Phe Glu Phe Pro Ile Gly Thr Tyr Leu Asn Tyr Glu Cys
            20                  25                  30

Arg Pro Gly Tyr Ser Gly Arg Pro Phe Ser Ile Ile Cys Leu Lys Asn
        35                  40                  45

Ser Val Trp Thr Gly Ala Lys Asp Arg Cys Arg Arg
    50                  55                  60

(2) INFORMATION FOR SEQ ID NO:2:
(i) SEQUENCE CHARACTERISTICS:
(A) LENGTH: 60 amino acids
(B) TYPE: amino acid
(C) STRANDEDNESS: single

(D) TOPOLOGY: linear
(ii) MOLECULE TYPE: protein
(iii) HYPOTHETICAL: NO
(iv) ANTI-SENSE: NO
(v) FRAGMENT TYPE: N-terminal
(xi) SEQUENCE DESCRIPTION: SEQ ID NO:2:

His Cys Gln Ala Pro Asp His Phe Leu Phe Ala Lys Leu Lys Thr Gln
1               5                   10                  15

Thr Asn Ala Ser Asp Phe Pro Ile Gly Thr Ser Leu Lys Tyr Glu Cys
            20                  25                  30

Arg Pro Glu Tyr Tyr Gly Arg Pro Phe Ser Ile Thr Cys Leu Asp Asn
        35                  40                  45

Leu Val Trp Ser Ser Pro Lys Asp Val Cys Lys Arg
    50                  55                  60

(2) INFORMATION FOR SEQ ID NO:3:
(i) SEQUENCE CHARACTERISTICS:
(A) LENGTH: 62 amino acids
(B) TYPE: amino acid
(C) STRANDEDNESS: single
(D) TOPOLOGY: linear
(ii) MOLECULE TYPE: protein
(iii) HYPOTHETICAL: NO
(iv) ANTI-SENSE: NO
(v) FRAGMENT TYPE: N-terminal
(xi) SEQUENCE DESCRIPTION: SEQ ID NO:3:

Lys Ser Cys Arg Asn Pro Pro Asp Pro Val Asn Gly Met Val His Val
1               5                   10                  15

Ile Lys Gly Ile Gln Phe Gly Ser Gln Ile Lys Tyr Ser Cys Thr Lys
            20                  25                  30

Gly Tyr Arg Leu Ile Gly Ser Ser Ser Ala Thr Cys Ile Ile Ser Gly
        35                  40                  45

Asp Thr Val Ile Trp Asp Asn Glu Thr Pro Ile Cys Asp Arg
    50                  55                  60

(2) INFORMATION FOR SEQ ID NO:4:
(i) SEQUENCE CHARACTERISTICS:
(A) LENGTH: 62 amino acids
(B) TYPE: amino acid
(C) STRANDEDNESS: single
(D) TOPOLOGY: linear
(ii) MOLECULE TYPE: protein
(iii) HYPOTHETICAL: NO
(iv) ANTI-SENSE: NO
(v) FRAGMENT TYPE: N-terminal
(xi) SEQUENCE DESCRIPTION: SEQ ID NO:4:

Lys Ser Cys Lys Thr Pro Pro Asp Pro Val Asn Gly Met Val His Val
1               5                   10                  15

Ile Thr Asp Ile Gln Val Gly Ser Arg Ile Thr Tyr Ser Cys Thr Thr
            20                  25                  30

Gly His Arg Leu Ile Gly His Ser Ser Ala Glu Cys Ile Leu Ser Gly
        35                  40                  45

Asn Ala Ala His Trp Ser Thr Lys Pro Pro Ile Cys Gln Arg
    50                  55                  60

(2) INFORMATION FOR SEQ ID NO:5:
(i) SEQUENCE CHARACTERISTICS:
(A) LENGTH: 8 amino acids
(B) TYPE: amino acid
(C) STRANDEDNESS: single
(D) TOPOLOGY: linear
(ii) MOLECULE TYPE: protein

(iii) HYPOTHETICAL: NO
(iv) ANTI-SENSE: NO
(v) FRAGMENT TYPE: N-terminal
(xi) SEQUENCE DESCRIPTION: SEQ ID NO:5:

Asp Lys Lys Ala Pro Ile Cys Asp
1               5

(2) INFORMATION FOR SEQ ID NO:6:
(i) SEQUENCE CHARACTERISTICS:
(A) LENGTH: 8 amino acids
(B) TYPE: amino acid
(C) STRANDEDNESS: single
(D) TOPOLOGY: linear
(ii) MOLECULE TYPE: protein
(iii) HYPOTHETICAL: NO
(iv) ANTI-SENSE: NO
(v) FRAGMENT TYPE: N-terminal
(xi) SEQUENCE DESCRIPTION: SEQ ID NO:6:

Ser Asp Pro Leu Pro Glu Cys Arg
1               5

(2) INFORMATION FOR SEQ ID NO:7:
(i) SEQUENCE CHARACTERISTICS:
(A) LENGTH: 4 amino acids
(B) TYPE: amino acid
(C) STRANDEDNESS: single
(D) TOPOLOGY: linear
(ii) MOLECULE TYPE: protein
(iii) HYPOTHETICAL: NO
(iv) ANTI-SENSE: NO
(v) FRAGMENT TYPE: N-terminal
(xi) SEQUENCE DESCRIPTION: SEQ ID NO:7:

Ser Ser Val Gln
1

(2) INFORMATION FOR SEQ ID NO:8:
(i) SEQUENCE CHARACTERISTICS:
(A) LENGTH: 12 amino acids
(B) TYPE: amino acid
(C) STRANDEDNESS: single
(D) TOPOLOGY: linear
(ii) MOLECULE TYPE: protein
(iii) HYPOTHETICAL: NO
(iv) ANTI-SENSE: NO
(v) FRAGMENT TYPE: N-terminal
(xi) SEQUENCE DESCRIPTION: SEQ ID NO:8:

Ser Asp Pro Leu Pro Glu Cys Arg Ser Ser Val Gln
1               5                   10

(2) INFORMATION FOR SEQ ID NO:9:
(i) SEQUENCE CHARACTERISTICS:
(A) LENGTH: 12 amino acids
(B) TYPE: amino acid
(C) STRANDEDNESS: single
(D) TOPOLOGY: linear
(ii) MOLECULE TYPE: protein
(iii) HYPOTHETICAL: NO
(iv) ANTI-SENSE: NO
(v) FRAGMENT TYPE: N-terminal
(xi) SEQUENCE DESCRIPTION: SEQ ID NO:9:

Ser Thr Lys Pro Pro Ile Cys Gln Asn Ala Ala His
1               5                   10

(2) INFORMATION FOR SEQ ID NO:10:
(i) SEQUENCE CHARACTERISTICS:
(A) LENGTH: 8 amino acids
(B) TYPE: amino acid
(C) STRANDEDNESS: single
(D) TOPOLOGY: linear
(ii) MOLECULE TYPE: protein

(iii) HYPOTHETICAL: NO
(iv) ANTI-SENSE: NO
(v) FRAGMENT TYPE: N-terminal
(xi) SEQUENCE DESCRIPTION: SEQ ID NO:10:

Gln Pro Tyr Ile Thr Gln Asn Tyr
1               5

(2) INFORMATION FOR SEQ ID NO:11:
(i) SEQUENCE CHARACTERISTICS:
(A) LENGTH: 43 amino acids
(B) TYPE: amino acid
(C) STRANDEDNESS: single
(D) TOPOLOGY: linear
(ii) MOLECULE TYPE: protein
(iii) HYPOTHETICAL: NO
(iv) ANTI-SENSE: NO
(v) FRAGMENT TYPE: N-terminal
(xi) SEQUENCE DESCRIPTION: SEQ ID NO:11:

Thr Arg Thr Thr Phe His Leu Gly Arg Lys Cys Ser Thr Ala Val Ser
1               5                   10                  15

Pro Ala Thr Thr Ser Glu Gly Leu Arg Leu Cys Ala Ala His Pro Arg
            20                  25                  30

Glu Thr Gly Ala Leu Gln Pro Pro His Val Lys
        35                  40